# FASCICULE DE BREVET EUROPEEN

(11) **EP 1 265 891 B1**
(45) Date de publication et mention de la délivrance du brevet: **29.12.2004**
(21) Numéro de dépôt: 01917143.8
(22) Date de dépôt: 15.03.2001
(51) Int. Cl.: C07D 409/12, C07D 339/04, C07D 333/38, A61K 31/385, A61P 9/00

(54) **NOUVEAUX DERIVES HETEROCYCLIQUES OU BENZENIQUES DE L'ACIDE LIPOIQUE, LEUR PREPARATION ET LEUR APPLICATION A TITRE DE MEDICAMENTS**
HETEROZYCLISCHE ODER BENZOL DERIVATE VON LIPONSÄURE, DEREN HERSTELLUNG UND DEREN VERWENDUNG ALS HEILMITTEL
NOVEL LIPOIC ACID HETEROCYCLIC OR BENZENE DERIVATIVES, PREPARATION AND USE THEREOF AS MEDICINES

(30) Priorité: 16.03.2000 FR 0003355; 21.09.2000 FR 0012007
(43) Date de publication de la demande: 18.12.2002
(73) Titulaire: SOCIETE DE CONSEILS DE RECHERCHES ET D'APPLICATIONS SCIENTIFIQUES (S.C.R.A.S.), 75016 Paris (FR)
(72) Inventeur: HARNETT, Jeremiah, F-91190 Gif-sur-Yvette (FR); AUGUET, Michel, F-91120 Palaiseau (FR)
(74) Mandataire: Bourgouin, André
(86) Numéro de dépôt international: PCT/FR2001/000764
(87) Numéro de publication internationale: WO 2001/068643

(56) Documents cités:
- EP-A- 0 798 292
- EP-A- 0 869 126
- WO-A-00/59899
- FR-A- 2 761 066
- FR-A- 2 801 053
- US-A- 6 030 985

## Description

La présente invention a pour objet des nouveaux dérivés hétérocycliques ou benzéniques comportant une chaîne latérale dérivée de l'acide lipoïque, lesquels présentent une activité inhibitrice des enzymes NO-synthases produisant le monoxyde d'azote NO et / ou sont des agents permettant la régénération d'antioxydants ou d'entités piégeuses des formes réactives de l'oxygène (ROS pour "*reactive oxygen species"*) et intervenant d'une façon plus générale dans le statut rédox des groupement thiols. Ces antioxydants ou entités piégeuses des formes réactives de l'oxygène peuvent être d'origine naturelle, comme par exemple la vitamine E ou le glutathion, ou d'origine synthétique comme certains produits piégeurs de ROS ou des produits présentant à la fois une activité inhibitrice des enzymes NO-synthases et une activité piégeuse de ROS. Des exemples de tels produits d'origine synthétique peuvent notamment être trouvés dans les demandes de brevet PCT WO 96/09653, WO 98/42696 et WO 98/58934.

L'invention concerne donc en particulier les dérivés correspondant à la formule générale **(I)** définie ci-après, leurs méthodes de préparation, les préparations pharmaceutiques les contenant et leur utilisation à des fins thérapeutiques, en particulier leur utilisation en tant qu'inhibiteurs des NO-synthases et / ou agents permettant la régénération d'antioxydants ou d'entités piégeuses des ROS et intervenant d'une façon plus générale dans le statut rédox des groupement thiols.

Compte tenu du rôle potentiel du NO, des ROS et du métabolisme du glutathion en physiopathologie, les nouveaux dérivés décrits répondant à la formule générale **(I)** peuvent produire des effets bénéfiques ou favorables dans le traitement de pathologies où le monoxyde d'azote et le métabolisme du glutathion ainsi que le statut rédox des groupement thiols sont impliqués, et notamment :
- troubles cardiovasculaires et cérébrovasculaires comprenant par exemple l'athérosclérose, la migraine, l'hypertension artérielle, le choc septique, les infarctus cardiaques ou cérébraux d'origine ischémique ou hémorragiques, les ischémies et les thromboses ;
- troubles du système nerveux central ou périphérique comme par exemple les maladies neurodégénératives où l'on peut notamment citer les infarctus cérébraux, l'hémorragie sub arachnoïde, le vieillissement, les démences séniles, y compris la maladie d'Alzheimer, la chorée de Huntington, la maladie de Parkinson, la maladie de Creutzfeld Jacob et les maladies à prions, la sclérose latérale amyotrophique mais aussi la douleur et la fibromyalgie, les traumatismes cérébraux ou de la moelle épinière, l'addiction aux opiacées, à l'alcool et aux substances induisant une accoutumance, les troubles de l'érection et de la reproduction, les désordres cognitifs, les encéphalopathies, les encéphalopathies d'origine virale ou toxique, la dépression, l'anxiété, la schizophrénie, l'épilepsie, les troubles du sommeil, les troubles alimentaires (anorexie, boulimie...) ;
- troubles du muscle squelettique et des jonctions neuromusculaires (myopathie, myosite) ainsi que les maladies cutanées ;
- les maladies prolifératives et inflammatoires comme par exemple l'athérosclérose, l'hypertension pulmonaire, la détresse respiratoire, la glomérulonéphrite, la cataracte, l'hypertension portale, le psoriasis, l'arthrose et l'arthrite rhumatoïde, les fibroses, les amyloïdoses, les inflammations du système gastro-intestinal (colite, maladie de Crohn) ou du système pulmonaire et des voies aériennes (asthme, sinusites, rhinites) ainsi que les hypersensibilités de contact ou retardées ;
- les transplantations d'organes ;
- les maladies auto-immunes et virales comme par exemple le lupus, le sida, les infections parasitaires et virales, le diabète et ses complications incluant les rétinopathies, les néphropathies et les polyneuropathies, la sclérose en plaques, les myopathies ;
- le cancer ;
- les maladies génétiques autosomiques comme la maladie d'Unverricht-Lunborg ;
- les maladies neurologiques associées à des intoxications (empoisonnement au Cadmium, inhalation de n-hexane, pesticide, herbicide), à des traitements (radiothérapie) ou à des désordres d'origine génétique (maladie de Wilson) ;
- l'impuissance liée au diabète ;
- toutes les pathologies caractérisées par une production ou un dysfonctionnement de monoxyde d'azote et/ou du métabolisme du glutathion et du statut rédox des groupement thiols.

Dans l'ensemble de ces pathologies, il existe des évidences expérimentales démontrant l'implication du monoxyde d'azote ou d'un dysfonctionnement du métabolisme du glutathion (Kerwin et al., Nitric oxide : a new paradigm for second messengers, *J*. *Med*. *Chem.* **38,** 4343-4362, 1995 ; Packer et al., Alpha-lipoic acid as biological antioxidant, *Free Radical Biology & Medicine* **19,** 227-250, 1995). C'est le cas notamment dans la maladie de Parkinson (Beal MF, Excitotoxicity and nitric oxide in Parkison's disease pathogenesis, *Ann. Neurol.* **44**[Suppl 1], S110-S114, 1998; Donato A et al., Gluthathione in Parkinson's disease: a link between oxidative stress and mitochondrial damage, *Ann. Neurol*. **32,** S111-S115, 1992). Dans ce contexte, les médicaments pouvant inhiber la formation du monoxyde d'azote ou rétablir la fonctionnalité biologique des groupements thiols ou du glutathion peuvent apporter des effets bénéfiques.

Par ailleurs les inventeurs ont déjà décrit dans des brevets antérieurs des inhibiteurs de NO Synthases et leur utilisation (brevets US 5,081,148 ; US 5,360,925), et plus récemment l'association de ces inhibiteurs avec des produits possédant des propriétés antioxydantes ou antiradicalaires (demande de brevet PCT WO 98/09653). Ils ont aussi décrit des dérivés d'amidines dans les demandes de brevet PCT WO 98/42696 et WO 98/58934 et des dérivés d'aminopyridines dans la demande de brevet PCT WO 00/02860. Ces dérivés d'amidines ou d'aminopyridines présentent la particularité d'être à la fois des inhibiteurs de NO Synthases et des inhibiteurs de ROS.

De plus, la demande de brevet EP 869 126 décrit, en tant qu'intensificateurs de l'activité de la glutathione réductase, des dérivés de l'acide lipoïque de formule générale 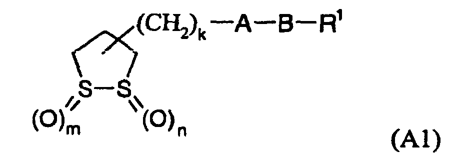 dans laquelle :
l'un de m et n est 0 et l'autre est 0, 1 ou 2,
k est 0 ou un entier de 1 à 12,
A est une simple liaison, un atome d'oxygène, un groupe carbonyle ou un groupe de formule -N(R²)CO-, -N(R²)CS-, -N(R²)SO₂-, -CON(R²)N(R³)CO-, -CON(R²)CO-, -CON(R²)CS-, -CON(R²)SO₂-, -O-CO-, -ON(R²)CO-, -ON(R²)SO₂-, -O-CON(R²)N(R³)CO-, -O-CON(R²)CO-, -O-CON(R²)SO₂-, -CO-O-, -CO-CO-, -CO-CON(R²)N(R³)CO-, -CO-CON(R²)CO-, -CO-CON(R²)SO₂-, -N(R²)O-, -N(R²)COCO-, -N(R²)N(R³)CO-, -N(R²)N(R²)SO₂-, -N(R²)CON(R³)N(R⁴)CO-, -N(R²)CON(R³)CO-, -N(R²)CON(R³)SO₂- ou -N(R²)CON(R³)SO₂N(R⁴)CO- dans laquelle R², R³ et R⁴ sont identiques ou différents et chacun est hydrogène, alkyle, aralkyle, acyle ou l'un des substituants α ;
B est une simple liaison, ou un groupe de formule -N(R⁵)- ou -N(R⁶)N(R⁵)- dans laquelle R⁵ et R⁶ sont identiques ou différents et chacun est hydrogène, alkyle, aralkyle, acyle ou l'un des substituants α, ou R⁵, ensemble avec R¹ et l'atome d'azote auquel ils sont liés, peuvent former un hétérocycle de 5 à 7 chaînons ;
ou R¹ peut représenter un groupe de formule -OR⁷, dans laquelle R⁷ est alkyle, alkényle, aralkyle ou l'un des substituants α ; ou encore R¹ peut représenter un groupe hydroxy ou un groupe de formule -OR⁷ ;
et lesdits substituants α sont choisis parmi des groupes aryle et des groupes hétérocycliques éventuellement substitués ;
ont été décrits dans la demande de brevet EP 869 126 en tant qu'intensificateurs de l'activité de la glutathione réductase.

Des dérivés du 2-(iminométhyl)aminophényle possédant au moins une activité d'inhibition des NO synthases sont par ailleurs décrits dans les demandes de brevet FR 2 761 066 et FR 2 801 053, le brevet US 6,030,985 ou la demande de brevet EP 798 292. Tous ces composés diffèrent de ceux de l'invention au moins par l'absence d'un motif dithiolane dans leur structure.

Enfin, la demande PCT WO 00/50899 concerne des composés de formule générale **(A2),** laquelle correspond aux sous-formules générales **(A2a)** et **(A2b)** 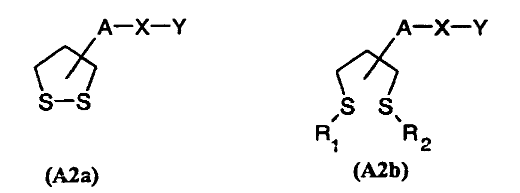 dans lesquelles
R₁ et R₂ représentent indépendamment H ou (C₁-C₆)-alkyle ;
A représente -(CH₂)ₘ-NR₃-CO(CH₂)ₙ-, -(CH₂)ₘ-CO-NR₃-(CH₂)ₙ-, -(CH₂)ₘ-NR₃-(CH₂)ₙ-, -(CH₂)ₘ-CO-NR₃-(CH₂)ₚ-NR₄-(CH₂)ₙ-, -(CH₂)ₘ-NR₃-CO-NR₄-(CH₂)ₙ- ou -(CH₂)ₘ-, m et n étant des entiers de 0 à 6 et p étant un entier de 2 à 6, et R₃ et R₄ représentant indépendamment H ou (C₁-C₆)-alkyle ;
X représente un radical 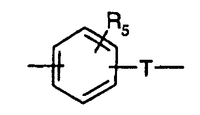
dans lequel le groupe T, qui est attaché au groupe Y, représente un radical -(CH₂)ᵢ- dans lequel i représente un entier compris entre 0 et 6, et R₅ représente H, (C₁-C₆)-alkyle ou -(CH₂)ₘ-Q dans lequel Q représente un atome halogène, ou hydroxy, cyano, amino, alkoxy, alkylthio, alkylamino ou dialkylamino, ou encore R₅ représente un hétérocycle de 5 à 6 chaînons dont les chaînons hétérocycliques sont choisis parmi ―O-, -N(R₆)- et -S-, R₆ représentant H, (C₁-C₆)-alkyle ou la liaison vers le cycle phényle du radical X ;
ou encore X représente un radical -(CH₂)_{q}- dans lequel q représente un entier de 0 à 6 ;
et enfin Y représente l'un des radicaux 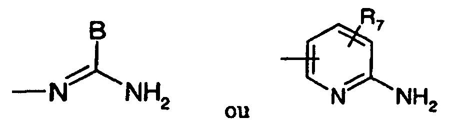 dans lesquels :
B représente un radical (C₁-C₆)-alkyle, aryle carbocyclique ou hétérocyclique à 5 ou 6 chaînons contenant de 1 à 4 hétéroatomes choisis parmi O, S et N, ledit radical aryle étant éventuellement substitué,
ou B représente NR₈R₉, dans lequel R₈ et R₉ représentent, indépendamment, H ou un radical (C₁-C₆)-alkyle, ou l'un de R₈ et R₉ représente un radical nitro tandis que l'autre représente H ou un radical (C₁-C₆)-alkyle, ou encore R₈ et R₉ pris ensemble forment avec l'atome d'azote un hétérocycle non aromatique de cinq à six chaînons, les éléments de la chaîne étant choisis parmi un groupe composé de -CH₂-, -NH-, -O- ou -S-,
ou encore B représente SR₁₀, R₁₀ représentant H ou un radical (C₁-C₆)-alkyle,
et R₇ représente H ou un radical (C₁-C₆)-alkyle.

La présente invention a pour objet de nouveaux dérivés hétérocycliques ou benzéniques comportant une chaîne latérale dérivée de l'acide lipoïque, leur préparation et leur application en thérapeutique.

L'invention concerne donc un produit de formule générale **(I)**, caractérisée en ce qu'elle comprend les produits de sous-formules **(I)a** et **(I)b** 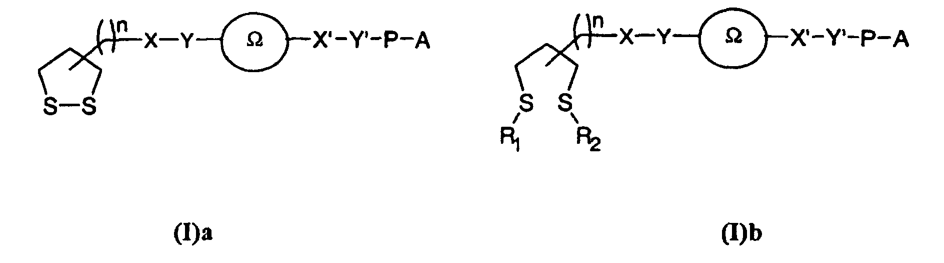 dans lesquelles
n représente un entier de 0 à 6 ;
R₁ et R₂ représentent, indépendamment, un atome d'hydrogène ou un radical alkyle linéaire ou ramifié ayant de 1 à 6 atomes de carbone ;
X-Y représente -O-(CH₂)ᵣ-, -N(R₃)-(CH₂)ᵣ-, -CO(CH₂)ᵣ-, -CO-N(R₃)-(CH₂)ᵣ-, -N(R₄)-CO-(CH₂)ᵣ-, ou -N(R₃)-CO-N(R₄)-(CH₂)ᵣ-,
X'-Y' représente -(CH₂)ᵣ-, -(CH₂)ᵣ-O-(CH₂)ᵣ-, -(CH₂)ᵣ-N(R₃)-(CH₂)ᵣ-, -(CH₂)ᵣ-CO(CH₂)ᵣ-, -(CH₂)ᵣ-CO-N(R₃)-(CH₂)ᵣ-, -(CH₂)ᵣ-N(R₄)-CO-(CH₂)ᵣ- ou -(CH₂)ᵣ-N(R₃)-CO-N(R₄)-(CH₂)ᵣ-,
R₃ et R₄ représentent, indépendamment à chaque fois qu'ils interviennent, un atome d'hydrogène ou un radical alkyle, alkoxycarbonyle ou aralkoxycarbonyle ;
r représentant indépendamment à chaque fois qu'il intervient un entier de 0 à 6 ;
Ω représente un hétérocycle aromatique à 5 ou 6 chaînons, un hétérocycle non aromatique de 4 à 7 chaînons ou un radical phénylène substitué par un radical R₅, R₅ représentant un atome d'hydrogène, un radical alkyle linéaire ou ramifié de 1 à 6 atomes de carbone ou un radical -(CH₂)ₘ-Q dans lequel Q représente un atome halogène ou un radical hydroxy, cyano, amino, alkoxy, alkylamino ou dialkylamino, m représentant un entier de 0 à 6 ;
P représente -(CH₂)_{g}-, g représentant un entier de 0 à 6, ou encore P représente un radical 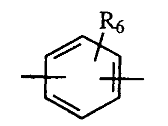
dans lequel R₆ représente un atome d'hydrogène, un radical alkyle linéaire ou ramifié ayant de 1 à 6 atomes de carbone ou un radical -(CH₂)ₙ-Q' dans lequel Q' représente un atome halogène ou un radical trifluorométhyle, hydroxy, amino, cyano, alkoxycarbonylamino, aralkoxycarbonylamino, alkoxy, alkylthio, alkylamino ou dialkylamino et n représente un entier de 0 à 6, ou encore R₆ représente un hétérocycle aromatique ou non aromatique de 5 à 6 chaînons dont les chaînons hétérocycliques sont choisis parmi les radicaux -O-, -N(R₇)- et -S-, R₇ représentant un atome d'hydrogène ou un alkyle linéaire ou ramifié ayant de 1 à 6 atomes de carbone ;
et enfin A représente le radical 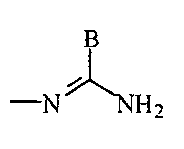 dans lequel :
B représente un radical alkyle linéaire ou ramifié ayant de 1 à 6 atomes de carbone, aryle carbocyclique ou hétérocyclique à 5 ou 6 chaînons contenant de 1 à 4 hétéroatomes choisis parmi O, S et N et notamment les radicaux thiophène, furanne, pyrrole ou thiazole, le radical aryle étant éventuellement substitué par un ou plusieurs groupes choisis parmi les radicaux alkyle, alkényle ou alkoxy linéaires ou ramifiés ayant de 1 à 6 atomes de carbone,
ou B représente NR₈R₉, dans lequel R₈ et R₉ représentent, indépendamment, un atome d'hydrogène ou un radical alkyle linéaire ou ramifié ayant de 1 à 6 atomes de carbone,
ou l'un de R₈ et R₉ représente un radical nitro tandis que l'autre représente un atome d'hydrogène ou un radical alkyle linéaire ou ramifié ayant de 1 à 6 atomes de carbone,
ou encore R₈ et R₉ pris ensemble forment avec l'atome d'azote un hétérocycle non aromatique de cinq à six chaînons, les éléments de la chaîne étant choisis parmi un groupe composé de -CH₂-, -NH-, -O- ou -S-,
ou encore B représente un radical SR₁₀ dans lequel R₁₀ représente un atome d'hydrogène ou un radical alkyle linéaire ou ramifié ayant de 1 à 6 atomes de carbone ;
ou un sel d'un produit de formule générale **(I)**.

Par alkyle, lorsqu'il n'est pas donné plus de précision, on entend un radical alkyle linéaire ou ramifié comptant de 1 à 6 atomes de carbone. Par cycloalkyle, lorsqu'il n'est pas donné plus de précision, on entend un système monocyclique carboné comptant de 3 à 7 atomes de carbone. Par alkényle, lorsqu'il n'est pas donné plus de précision, on entend un radical alkyle linéaire ou ramifié comptant de 1 à 6 atomes de carbone et présentant au moins une insaturation (double liaison). Par alkynyle, lorsqu'il n'est pas donné plus de précision, on entend un radical alkyle linéaire ou ramifié comptant de 1 à 6 atomes de carbone et présentant au moins une double insaturation (triple liaison). Par allènyle, on entend le radical -CH=C=CH₂. Par aryle carbocyclique ou hétérocyclique, on entend un système carbocyclique (en particulier, le radical phényle qui peut être noté de façon abrégée Ph) ou hétérocyclique comprenant au moins un cycle aromatique, un système étant dit hétérocyclique lorsque l'un au moins des cycles qui le composent comporte un hétéroatome (O, N ou S). Par hétérocycle, lorsqu'il n'est pas donné plus de précision, on entend un système mono- ou polycyclique ledit système comprenant au moins un hétéroatome choisi parmi O, N et S et étant saturé, partiellement ou totalement insaturé ou aromatique. Par hétéroaryle, lorsqu'il n'est pas donné plus de précision, on entend un hétérocycle tel que défini précédemment dans lequel l'un au moins des cycles qui le composent est aromatique. Par haloalkyle, on entend un radical alkyle dont au moins l'un des atomes d'hydrogène (et éventuellement tous) est remplacé par un atome halogène.

Par radicaux alkylthio, alkoxy, haloalkyle, alkoxyalkyle, trifluorométhylalkyle, cycloalkylalkyle, haloalkoxy, aminoalkyle, alkényle, alkynyle, allènylalkyle, cyanoalkyle et aralkyle, on entend respectivement les radicaux alkylthio, alkoxy, haloalkyle, alkoxyalkyle, trifluorométhylalkyle, cycloalkylalkyle, haloalkoxy, aminoalkyle, alkényle, alkynyle, allènylalkyle, cyanoalkyle et aralkyle dont le radical alkyle (les radicaux alkyle) a (ont) la (les) signification(s) indiquée(s) précédemment.

Par hétérocycle, on entend notamment les radicaux thiényle, pipéridyle, pipérazinyle, quinolinyle, indolinyle et indolyle. Par alkyle linéaire ou ramifié ayant de 1 à 6 atomes de carbone, on entend en particulier les radicaux méthyle, éthyle, propyle, isopropyle, butyle, isobutyle, sec-butyle et tert-butyle, pentyle, néopentyle, isopentyle, hexyle, isohexyle. Enfin, par halogène, on entend les atomes de fluor, de chlore, de brome ou d'iode.

De préférence, l'invention concerne les composés de formule générale **(I)** dans lesquels se retrouvent au moins une des caractéristiques suivantes :
- n représente un entier de 1 à 6 ;
- X-Y représente l'un des radicaux -O-(CH₂)ᵣ-, -N(R₃)-(CH₂)ᵣ-, -CO(CH₂)ᵣ-, -CO-N(R₃)-(CH₂)ᵣ-, -N(R₄)-CO-(CH₂)ᵣ-, ou -N(R₃)-CO-N(R₄)-(CH₂)ᵣ-, dans lesquels R₃ et R₄ représentent un atome d'hydrogène ou un radical alkyle ;
- X'-Y' représente l'un des radicaux -(CH₂)ᵣ-, -(CH₂)ᵣ-O-(CH₂)ᵣ-, -(CH₂)ᵣ-N(R₃)-(CH₂)ᵣ-, -(CH₂)ᵣ-CO(CH₂)ᵣ-, -(CH₂)ᵣ-CO-N(R₃)-(CH₂)ᵣ-, -(CH₂)ᵣ-N(R₄)-CO-(CH₂)ᵣ- ou -(CH₂)ᵣ-N(R₃)-CO-N(R₄)-(CH₂)ᵣ-, dans lesquels R₃ et R₄ représentent un atome d'hydrogène ou un radical alkyle ou alkoxycarbonyle ;
- Ω représente un radical phénylène substitué par un radical R₅, R₅ représentant un atome d'hydrogène, un radical alkyle linéaire ou ramifié de 1 à 6 atomes de carbone ou un radical -(CH₂)ₘ-Q dans lequel Q représente un atome halogène ou un radical cyano, amino ou alkoxy, m représentant un entier de 0 à 6, ou encore Ω représente un hétérocycle aromatique à 5 ou 6 chaînons ou un hétérocycle non aromatique de 4 à 7 chaînons, ledit hétérocycle aromatique ou non aromatique comprenant de préférence au moins un atome d'azote ;
- P représente un radical 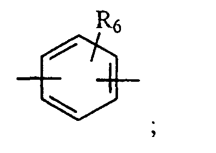
- A représente un radical 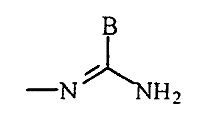
dans lequel B représente un radical aryle carbocyclique ou hétérocyclique à 5 ou 6 chaînons contenant de 1 à 4 hétéroatomes choisis parmi O, S et N.

Plus préférentiellement, l'invention concerne les composés de formule générale **(I)** dans lesquels se retrouvent au moins une des caractéristiques suivantes :
- n représente un entier de 1 à 5 ;
- X-Y représente l'un des radicaux -O-(CH₂)ᵣ-, -N(R₃)-(CH₂)ᵣ-, -CO(CH₂)ᵣ-, -CO-N(R₃)-(CH₂)ᵣ-, -N(R₄)-CO-(CH₂)ᵣ-, ou -N(R₃)-CO-N(R₄)-(CH₂)ᵣ-, dans lesquels R₃ et R₄ représentent un atome d'hydrogène ;
- X'-Y' représente l'un des radicaux -(CH₂)ᵣ-, -(CH₂)ᵣ-O-(CH₂)ᵣ-, -(CH₂)ᵣ-N(R₃)-(CH₂)ᵣ-, -(CH₂)ᵣ-CO(CH₂)ᵣ-, -(CH₂)ᵣ-CO-N(R₃)-(CH₂)ᵣ-, -(CH₂)ᵣ-N(R₄)-CO-(CH₂)ᵣ- ou -(CH₂)ᵣ-N(R₃)-CO-N(R₄)-(CH₂)ᵣ-, dans lesquels R₃ et R₄ représentent un atome d'hydrogène ;
- Ω représente un radical phénylène substitué par un radical R₅, R₅ représentant un atome d'hydrogène, un radical alkyle linéaire ou ramifié de 1 à 6 atomes de carbone ou un radical -(CH₂)ₘ-Q dans lequel Q représente un radical cyano ou alkoxy, ou encore Ω représente un hétérocycle non aromatique de 4 à 7 chaînons comprenant de préférence au moins un atome d'azote ;
- P représente un radical 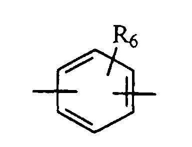 dans lequel R₆ représente un radical -(CH₂)ₙ-Q' dans lequel Q' représente un atome d'hydrogène ou un radical alkoxy, cyano, amino, alkylamino ou dialkylamino et n représente un entier de 0 à 6 et de préférence un entier de 0 à 3 ;
- A représente un radical 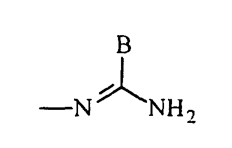
dans lequel B représente un radical aryle hétérocyclique à 5 ou 6 chaînons contenant de 1 à 4 hétéroatomes choisis parmi O, S et N.

Encore plus préférentiellement, l'invention concerne les composés de formule générale **(I)** dans lesquels se retrouvent au moins une des caractéristiques suivantes :
- n représente un entier de 1 à 4 ;
- X-Y représente l'un des radicaux -CO(CH₂)ᵣ-, -CO-N(R₃)-(CH₂)ᵣ- ou -N(R₄)-CO-(CH₂)ᵣ-, dans lesquels R₃ et R₄ représentent un atome d'hydrogène ;
- X'-Y' représente l'un des radicaux -(CH₂)ᵣ- ou -(CH₂)ᵣ-N(R₃)-(CH₂)ᵣ- ou -(CH₂)ᵣ-CO-N(R₃)-(CH₂)ᵣ-, dans lesquels R₃ et R₄ représentent un atome d'hydrogène ;
- Ω représente un radical pipérazinyle, pipéridyle ou phényle, et de préférence pipérazinyle ou pipéridyle ;
- P représente un radical 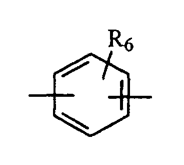 dans lequel R₆ représente un radical -(CH₂)ₙ-Q' dans lequel Q' représente un atome d'hydrogène ou un radical alkoxy, cyano ou amino et n représente un entier de 0 à 3 ;
- A représente un radical 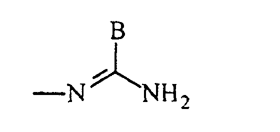
dans lequel B représente un radical thiényle, furannyle ou pyrrolyle.

Par ailleurs, on préférera d'une façon générale parmi les composés de formule générale **(I)** les composés répondant à la sous-formule générale **(I)a**.

Plus particulièrement, l'invention concerne les produits suivants décrits dans les exemples (parfois sous forme de sels) :
- N'-(4-{4-[5-(1,2-dithiolan-3-yl)pentanoyl]-1-pipérazinyl}phényl)-2-thiophènecarboximidamide ;
- N'-(4-{4-[2-(1,2-dithiolan-3-yl)acétyl]-1-pipérazinyl}phényl)-2-thiophènecarboximidamide ;
- N'-[3-({4-[5-(1,2-dithiolan-3-yl)pentanoyl]-1-pipérazinyl}méthyl)phényl]-2-thiophènecarboximidamide ;
- N'-[3-({4-[2-(1,2-dithiolan-3-yl)acétyl]-1-pipérazinyl}méthyl)phényl]-2-thiophènecarboximidamide ;
- 4-(4-{[amino(2-thiényl)méthylidène]amino}phényl)-N-[4-(1,2-dithiolan-3-yl)butyl]-1-pipérazinecarboxamide ;
- 4-(4-{[amino(2-thiényl)méthylidene]amino}-2-méthylphényl)-N-[4-(1,2-dithiolan-3-yl)butyl]-1-pipérazinecarboxamide ;
- 4-(4-{[amino(2-thiényl)méthylidène]amino}-3-méthylphényl)-N-[4-(1,2-dithiolan-3-yl)butyl]-1-pipérazinecarboxamide ;
- 4-(4-{[amino(2-thiényl)méthylidène]amino}-2-méthoxyphényl)-N-[4-(1,2-dithiolan-3-yl)butyl]-1-pipérazinecarboxamide ;
- 4-(4-{[amino(2-thiényl)méthylidène]amino}-3-méthoxyphényl)-N-[4-(1,2-dithiolan-3-yl)butyl]-1-pipérazinecarboxamide ;
- 4-(4-{[amino(2-thiényl)méthylidène]amino}-2-cyanophényl)-N-[4-(1,2-dithiolan-3-yl)butyl]-1-pipérazinecarboxamide ;
- 4-(3-{[amino(2-thiényl)méthylidène]amino}benzyl)-N-[4-(1,2-dithiolan-3-yl)butyl]-1-pipérazinecarboxamide ;
- N'-(3-{4-[5-(1,2-dithiolan-3-yl)pentanoyl]-1-pipérazinyl}phényl)-2-thiophènecarboximidamide ;
- 5-{[amino(2-thiényl)méthylidéne]amino}-2-{4-[5-(1,2-dithiolan-3-yl)pentanoyl]-1-pipérazinyl}benzylcarbamate de *tert*-butyle ;
- N'-(3-(aminométhyl)-4-{4-[5-(1,2-dithiolan-3-yl)pentanoyl]-1-pipérazinyl}phényl)-2-thiophènecarboximidamide ;
- 3-{[-amino(2-thiényl)méthylidène]amino}benzyl{1-[5-(1,2-dithiolan-3-yl)pentanoyl]-4-pipéridinyl}carbamate de tert-butyle ;
- N'-{3-[({1-[5-(1,2-dithiolan-3-yl)pentanoyl]-4-pipéridinyl}amino)méthyl]phényl}-2-thiophènecarboximidamide ;
- N-[2-(4-{[amino(2-thiényl)méthylidène]amino}phényl)éthyl]-4-{[5-(1,2-dithiolan-3-yl)pentanoyl]amino}benzamide ;
et leurs sels, notamment les chlorhydrates correspondants.

L'invention offre de plus un certain nombre de procédés pour accéder aux produits de formule générale **(I)** décrits ci-dessus, procédés dont les conditions préférées sont décrites plus loin.

L'invention concerne donc en particulier un procédé de préparation d'un composé de formule générale **(I)** telle que définie précédemment, caractérisé en ce que l'on fait réagir un composé de formule générale **(II)** 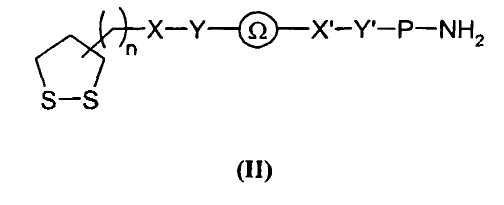 dans laquelle n, X, Y, Ω, X', Y' et P ont la même signification que dans la formule générale **(I)**,
a) avec le composé de formule générale **(I.i)** 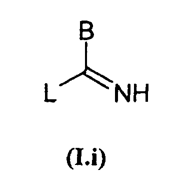 dans laquelle B a la même signification que dans la formule générale **(I)** et L représente un groupe partant, par exemple un radical alkoxy, alkylthio, acide sulfonique, halogénure, alcool arylique ou tosyle,
b) ou avec le composé de formule générale **(I.ii)** 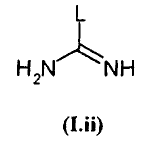 dans laquelle L représente un groupe partant, par exemple un radical alkoxy, alkylthio, acide sulfonique, halogénure, alcool arylique ou tosyle,
c) ou avec le composé de formule générale **(I.iii)** 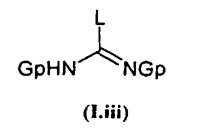 dans laquelle L représente un groupe partant, par exemple un radical alkoxy, alkylthio, acide sulfonique, halogénure, alcool arylique ou tosyle, et Gp un groupe protecteur de type carbamate, par exemple le groupe t-butoxycarbonyle,
   cette réaction étant suivie, dans le cas où l'on opte pour la réaction avec le composé de formule générale **(I.iii),** par une hydrolyse en présence d'un acide fort, par exemple l'acide trifluoroacétique,
d) ou avec le dérivé de formule **(I.iv)** (N-méthyl-N'-nitro-N-nitrosoguanidine) 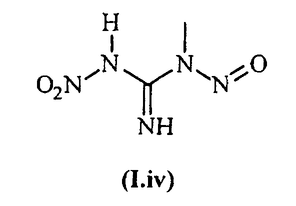
e) ou enfin avec le dérivé de formule **(I.v)** dans laquelle Gp représente un groupe protecteur

   S=·=N-Gp (I.v)

L'invention concerne également un procédé de préparation d'un produit de formule générale **(I)** dans laquelle le groupe X-Y représente -CO-N(R₃)-(CH₂)ᵣ-, caractérisé en ce que l'on fait réagir l'acide de formule générale **(I.vi)** 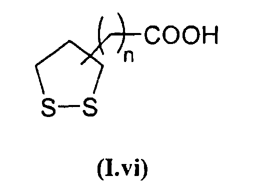 dans laquelle n a la même signification que dans la formule générale **(I),** avec une amine de formule générale **(X)** ou **(X*bis*)** 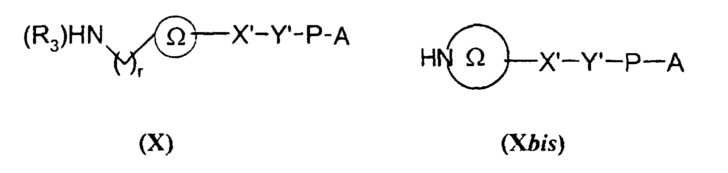 dans lesquelles r, R₃, Ω, X', Y', P et A ont la même signification que dans la formule générale **(I),** le composé de formule générale **(X*bis*)** étant en outre tel que son hétérocycle Ω comprend un atome d'azote.

L'invention a également pour objet, à titre de produits nouveaux, des intermédiaires de synthèse utiles pour la préparation de composés de formule générale **(I)**, à savoir les composés de formules générales **(II), (III), (IV), (X), (X*bis)*, (XI)** et **(XI*bis*)** telles que définies ci-dessus pour **(II), (X)** et **(X*bis*),** et plus loin pour **(III), (IV), (XI)** et **(XI*bis*)**.

Dans certains cas, les composés selon la présente invention peuvent comporter des atomes de carbone asymétriques, et donc posséder deux formes énantiomères possibles, c'est-à-dire les configurations "R" et "S". La présente invention inclut les deux formes énantiomères et toutes combinaisons de ces formes, y compris les mélanges racémiques "RS". Dans un souci de simplicité, lorsqu'aucune configuration spécifique n'est indiquée dans les formules de structure, il faut comprendre que les deux formes énantiomères et leurs mélanges sont représentés.

L'invention a également pour objet, à titre de médicaments, les composés décrits précédemment ou leurs sels pharmaceutiquement acceptables. Elle concerne aussi des compositions pharmaceutiques contenant ces composés ou leurs sels pharmaceutiquement acceptables, et l'utilisation de ces composés ou de leurs sels pharmaceutiquement acceptables pour fabriquer des médicaments destinés à inhiber la NO synthase neuronale ou la NO synthase inductible, à régénérer des anti-oxydants, lesquels peuvent être naturels ou synthétiques) ou à assurer la double fonction d'inhibition de la NO synthase et de régénération d'anti-oxydants.

Par sel pharmaceutiquement acceptable, on entend notamment des sels d'addition d'acides inorganiques tels que chlorhydrate, bromhydrate, iodhydrate, sulfate, phosphate, diphosphate et nitrate ou d'acides organiques tels que acétate, maléate, fumarate, tartrate, succinate, citrate, lactate, méthanesulfonate, p-toluènesulfonate, pamoate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Salt selection for basic drugs", *Int*. *J*. *Pharm*. (1986), **33,** 201-217.

L'invention a aussi pour objet l'utilisation d'un produit de formule générale **(I)** ou d'un sel pharmaceutiquement acceptable de ce produit pour préparer un médicament destiné à traiter des pathologies dans lesquelles le monoxyde d'azote et / ou le statut rédox des groupements thiols sont impliqués, pathologies telles que les troubles du système nerveux central ou périphérique particulièrement bien représentés par la maladie de Parkinson, les troubles cérébrovasculaires, les maladies prolifératives et inflammatoires, les vomissements, le choc septique, les pathologies résultant des irradiations radioactives, des radiations solaires ou des transplantations d'organes, les maladies autoimmunes et autosomales, les cancer et toutes les pathologies caractérisées par une production ou un dysfonctionnement impliquant le monoxyde d'azote et/ou impliquant le statut rédox des groupements thiols.

L'invention a encore pour objet l'utilisation d'un produit de formule générale **(I)** ou d'un sel pharmaceutiquement acceptable de ce produit, pour fabriquer un médicament destiné à traiter des troubles cérébrovasculaires tels que la migraine, les infarctus cérébraux d'origine ischémique ou hémorragique, les ischémies et les thromboses.

L'invention a enfin pour objet l'utilisation d'un produit de formule générale **(I)** ou d'un sel pharmaceutiquement acceptable de ce produit pour fabriquer un médicament destiné à traiter les troubles du système nerveux central ou périphériques tels que les maladies neurodégénératives, la douleur et la fibromyalgie, les traumatismes cérébraux ou de la moëlle épinière, le diabète et ses complications incluant les rétinopathies, les néphropathies et les polyneuropathies, l'addiction aux drogues opiacées, à l'alcool et aux substances induisant une accoutumance, les troubles de l'érection et de la reproduction, les désordres cognitifs, les encéphalopathies, la dépression , l'anxiété, la schizophrénie, l'épilepsie, les troubles du sommeil et les troubles alimentaires.

Les compositions pharmaceutiques peuvent être sous forme d'un solide, par exemple des poudres, des granules, des comprimés, des gélules, des liposomes ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

L'administration d'un médicament selon l'invention pourra se faire par voie topique, orale, parentérale, par injection intramusculaire, etc.

La dose d'administration envisagée pour médicament selon l'invention est comprise entre 0,1 mg à 10 g suivant le type de composé actif utilisé.

Conformément à l'invention, on peut préparer les composés de formule générale **(I)** par les procédés ci-après.

### PREPARATION DES COMPOSES DE L'INVENTION

### A) PREMIERE APPROCHE

Les composés de formule générale **(I)** peuvent être préparés à partir des intermédiaires de formule générale **(II), (III)** et **(IV)** selon le schéma 1 où n, X, X', Y, Y', Ω, P et A sont tels que définis ci-dessus et Gp est un groupe protecteur de type carbamate.

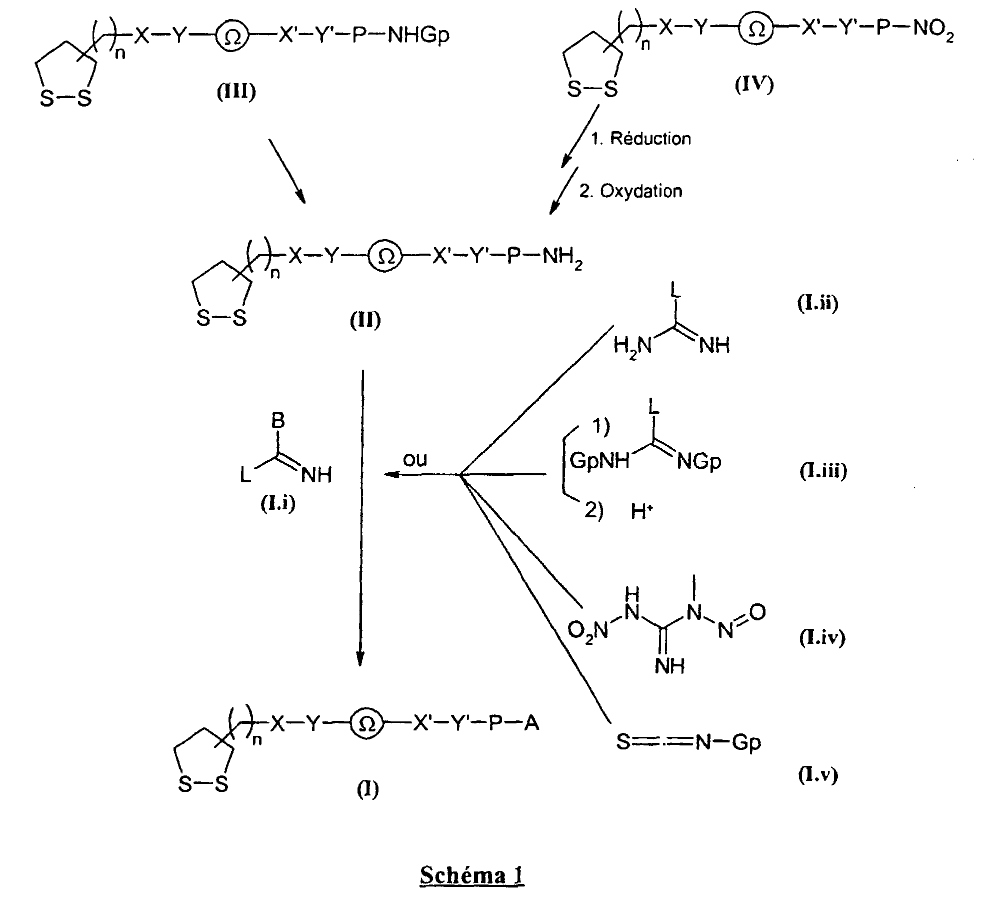

Les dérivés d'aniline et d'amines de formule générale **(II)**, peuvent être condensés sur des composés de formule générale **(I.i)**, dans lesquels L représente un groupe partant (en particulier un radical alkoxy, thioalkyle, acide sulfonique, halogénure, alcool arylique ou tosyle), pour conduire aux composés finaux de formule générale **(I)** de type amidine substitué (cf. schéma 1). Par exemple, pour B = thiophène, on peut condenser les dérivés de formule générale **(II)** sur l'iodhydrate de S-méthylthiophène thiocarboxamide, préparé selon une méthode de la littérature (*Ann*. *Chim*. (1962), **7**, 303-337). La condensation peut s'effectuer par chauffage dans un alcool (par exemple dans du méthanol ou de l'isopropanol), éventuellement en présence de DMF à une température de préférence comprise entre 50 et 100 °C pour une durée généralement comprise entre quelques heures et une nuit.

Dans les cas où B = SR₁₀, par exemple S-CH₃, ceux-ci peuvent être préparés par la condensation des amines ou anilines de formule générale **(II)** avec l'isothiocyanate **(I.v)** dans laquelle Gp représente un groupe protecteur tel que par exemple le groupe benzoyle. On déprotége ensuite par clivage du groupe protecteur dans des conditions convenables et la thiourée formée est finalement traitée avec, par exemple, un halogéno-alkane pour conduire aux composés finaux de formule générale **(I)**.

Dans les cas où B = NR₈R₉, les composés finaux de formule générale **(I)** sont des guanidines. Celles-ci peuvent être préparées, par exemple, par la condensation des amines ou anilines de formule générale **(II)** avec les dérivés de formule générale **(I.ii)** ou **(I.iii)**. Les réactifs de formule générale **(I.ii)** dans lesquels L représente, par exemple, un cycle pyrazole sont condensés sur les amines de formule générale **(II)** selon les conditions décrites dans la littérature (*J*. *Org. Chem*. (1992) **57,** 2497-2502). De même pour les réactifs de formule générale **(I.iii)** dans lesquels L représente, par exemple, un cycle pyrazole et Gp le groupement tBuOCO (*Tetrahedron Lett.* (1993) **34** (21), 3389-3392) ou bien lorsque L représente le groupement -N-SO₂-CF₃ et Gp le groupement tBuOCO (*J. Org*. *Chem*. (1998) **63,** 3804-3805). Lors de l'ultime étape de la synthèse, la déprotection de la fonction guanidine est effectuée en présence d'un acide fort tel que par exemple l'acide trifluoroacétique.

Dans les cas où B = -NHNO₂ les composés finaux de formule générale **(I)** peuvent être préparés, par exemple, par la condensation des amines ou anilines de formule générale **(II)** avec le réactif de formule **(I.iv)** (N-méthyl-N'-nitro-N-nitrosoguanidine) selon les conditions décrites dans la littérature ( J. Amer. Chem. Soc. (1947), **69,** 3028-3030 ).

Les composés de formule générale **(I)b,** sont obtenus à partir des composés de formule générale **(I)a** où n, X, X', Y, Y', Ω, P et A sont tels que définis ci-dessus. La transformation des composés lipoïques de formule générale **(I)a** en les dérivés dihydrolipoïques correspondants **(I)b** où R₁ = R₂ = H est effectuée dans un solvant alcoolique tel que, par exemple, le méthanol, en présence d'un agent réducteur tel que par exemple NaBH₄, NaBH₃CN ou l'hydrure d'aluminium et de lithium (LAH). Les composés pour lesquels R₁ et R₂ ne représentent pas tous deux H sont préparés en faisant réagir les composés de formule générale **(I)b** avec un composé de formule R₁- halogène et / ou R₂-halogène où R₁ et R₂ sont tels que définis ci-dessus et l'atome halogène est un groupe partant. La réaction est effectuée, par exemple, dans un solvant approprié tel que le THF, l'acétone, l'acétate d'éthyle en présence d'une base comme K₂CO₃ ou la triéthylamine, pour conduire aux composés de formule générale **(I)b**.

### Préparation des composés de formule générale (II) :

Les intermédiaires de formule générale **(II)**, sont obtenus à partir de la coupure d'un groupe protecteur (Gp) ou par réduction d'un groupement nitro.

Les intermédiaires de formule générale **(II)**, dans lesquels n, X, X', Y, Y', Ω, et P sont tels que définis ci-dessus, peuvent être préparés à partir des intermédiaires de formule générale **(III)** ou **(IV)**, schéma 1, qui sont des composés comportant respectivement une amine ou aniline protégée (NHGp) sous forme, par exemple, d'un carbamate ou un groupement nitro. Dans le cas particulier des groupes BOC, ceux-ci sont déprotégés classiquement à l'aide de TFA ou HCl , pour conduire finalement aux amines et anilines primaires de formule générale **(II)**. La réduction de la fonction nitro des intermédiaires de formule générale **(IV)**, schéma 1, dans lesquels n, X, X', Y, Y', Ω, et P sont tels que définis ci-dessus, est effectuée, par exemple, en chauffant le produit dans un solvant approprié tel que l'acétate d'éthyle avec un peu d'éthanol en présence de SnCl₂ (*J*. *Heterocyclic Chem.* (1987), 24, 927-930 ; *Tetrahedron Letters* (1984), 25 (8), 839-842) en présence de SnCl₂ / Zn (*Synthesis.* (1996), 9, 1076-1078), à l'aide de NaBH₄-BiCl₃ (*Synth*. *Com.* (1995) 25 (23), 3799-3803) dans un solvant tel que l'éthanol, ou alors en utilisant du Ni Raney additionné d'hydrate d'hydrazine *(Monatshefte für Chemie,* (1995), 126, 725-732), ou bien à l'aide d'indium dans un mélange d'éthanol et de chlorure d'ammonium à reflux (*Synlett (1998) 9*, 1028). Ensuite le produit de double réduction est ré-oxydé en présence de chlorure ferrique (FeCl₃) (*Synlett (1991)* **10,** *717-718* ) ou d'iode *(Tetrahedron Letters.*(1997), **38** (33), 5785-5788 ) pour conduire finalement aux amines et anilines de formule générale **(II)** contenant de nouveau le motif dithiolane.

### Préparation des composés de formule générale (III) et (IV):

### Synthèse des carboxamides de formule générale (III) et (IV) :

Les carboxamides de formule générale **(III)** et **(IV)** dans lesquels n, X, Y, X', Y', Ω et P sont tels que définis ci-dessus, lesquels peuvent posséder soit une fonction amide dont l'azote appartient à l'hétérocycle Ω, soit en une fonction amide dont l'azote est fixé au radical Ω ou à une chaîne alkylène liée à ce radical, schéma 2, sont préparés par condensation des acides de formule générale **(I.vi)** avec les amines ou anilines mono-protégées de formule générale **(VI)** et **(VIII)** ou les dérivés nitro de formule générale **(VII)** et **(IX)**. Les liaisons carboxamides sont formées dans des conditions classiques de synthèse peptidique (M. Bodanszky et A. Bodanszky, *The Practice of Peptide Synthesis,* 145 (Springer-Verlag, 1984)) dans le THF, le dichlorométhane ou le DMF en présence d'un réactif de couplage tel que le dicyclohexylcarbodiimide (DCC), le 1,1'-carbonyldiimidazole (CDI) (*J*. *Med. Chem.* (1992), **35** (23), 4464-4472) ou le chlorhydrate de 1-(3-diméthylaminopropyl)-3-éthylcarbodiimide (EDC ou WSCI) (John Jones, *The chemical synthesis of peptides,* **54** (Clarendon Press, Oxford, 1991)) ou en présence de chloroformiate d'isobutyle et de N-méthyl morpholine (*Org*. *Prep. Proced. Int*., (1975), 35, 215). Les synthèses des acides carboxyliques de formule générale **(I.vi)** et des amines / anilines de formule générale **(VI)**, **(VII)**, **(VIII)** et **(IX)** non commerciaux, sont décrites plus loin.

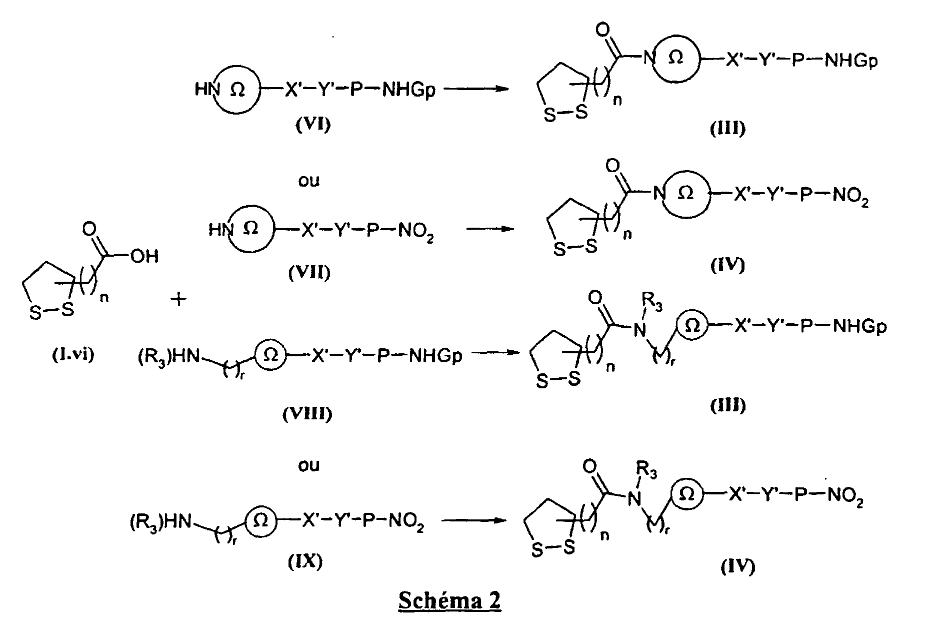

### B) DEUXIEME APPROCHE

Les composés de formule générale **(I)** peuvent aussi être préparés à partir des intermédiaires de formule générale **(X), (XI), (XII)** et **(XIII)** (ou **(X*bis*), (XI*bis*), (XII*bis*)** et **(XIII*bis*)** lorsque la fonction amine fait partie de l'hétérocycle Ω) selon le schéma 3 dans lequel n, X, X', Y, Y', Ω, P et A sont tels que définis ci-dessus et Gp₁ et Gp₂ sont des groupe protecteurs : par exemple, quand Y = -NR₃-, Gp₁ peut être un groupe protecteur de type carbamate ou quand Y = -O-, Gp₁ peut être un groupe protecteur de type benzyle ou d'autres groupes protecteurs (Gp₁) connus de l'homme du métier. Gp₂ peut aussi être un groupe protecteur de type carbamate ou d'autres groupes protecteurs (Gp₂) connus de l'homme du métier.

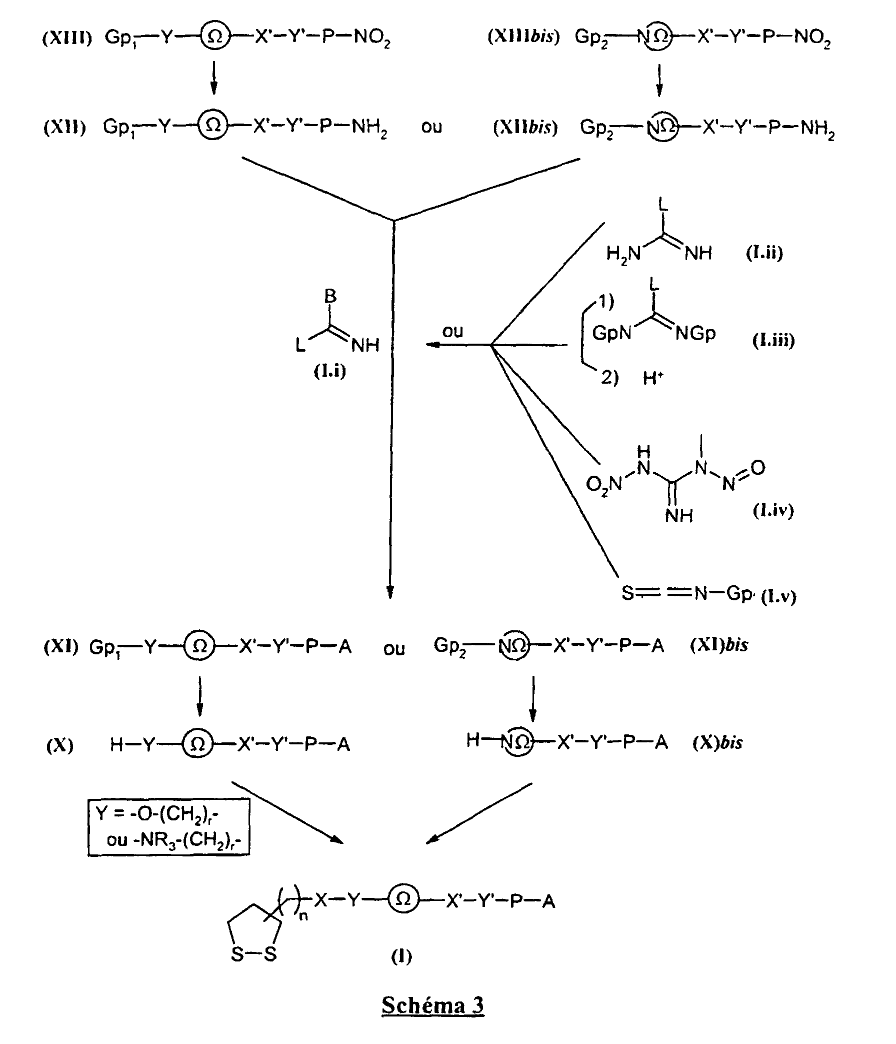

### Préparation des composés de formule générale (I) :

### Synthèse des carboxamides de formule générale (I) :

Les carboxamides de formule générale **(I),** schéma 4, dans lesquels n, X',Y', Ω, P et A sont tels que définis ci-dessus, sont préparés par condensation des acides de formule générale **(I.vi)** avec les amines / anilines de formule générale **(X)** et **(X)*bis***. Les liaisons carboxamides sont formées dans des conditions classiques de synthèse peptidique (M. Bodanszky et A. Bodanszky, *The Practice of Peptide Synthesis*, 145 (Springer-Verlag, 1984)) dans le THF, le dichlorométhane ou le DMF, en présence d'un réactif de couplage tel que le dicyclohexylcarbodiimide (DCC), le 1,1'-carbonyldiimidazole (CDI) (*J*. *Med*. *Chem*. (1992), 35 (23), 4464-4472) ou le chlorhydrate de 1-(3-diméthylaminopropyl)-3-éthylcarbodiimide (EDC ou WSCI) (John Jones, The chemical synthesis of peptides, 54 (Clarendon Press, Oxford, 1991)) ou en présence de chloroformiate d'isobutyle et de N-méthyl morpholine (*Org*. *Prep. Proced. Int*., (1975), 35, 215). Les synthèses des acides carboxyliques de forrnule générale **(I.vi)** non commerciaux, sont décrites plus loin.

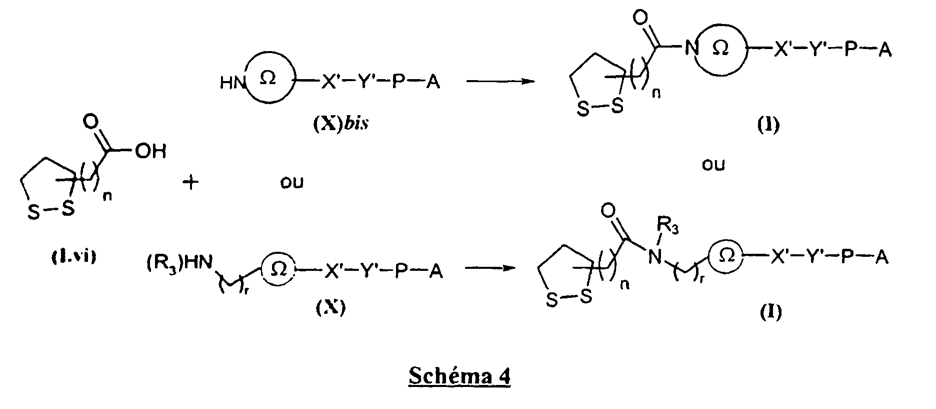

### Synthèse des urées de formule générale (I)

Les urées de formule générale **(I)**, schéma 5, dans lesquelles n, X',Y', Ω, P et A sont tels que définis ci-dessus, sont préparées, par exemple, par condensation des acides de formule générale **(I.vi)** avec les amines / anilines de formule générale **(X)** et **(X)*bis*** dans du toluène en présence de diphénylphosphoryl azide (DPPA) et de triéthylamine, par exemple à une température de 80° C et pendant 2 à 3 heures.

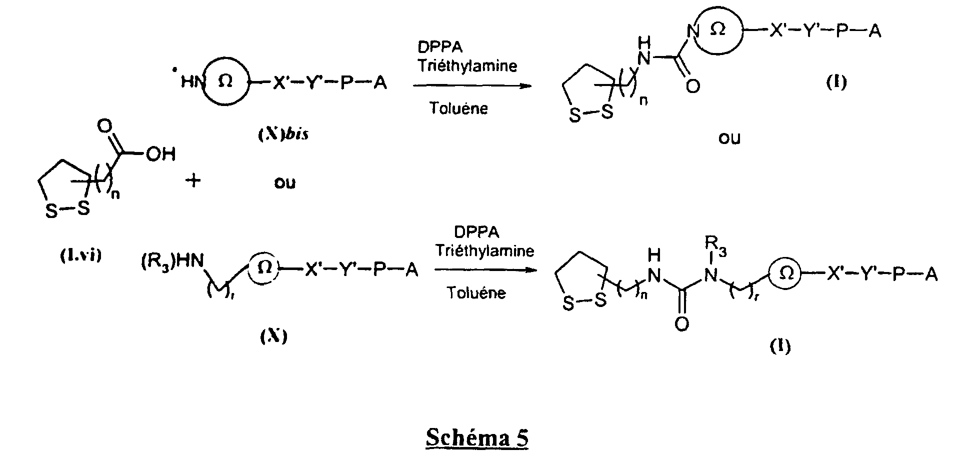

### Préparation des composés de formule générale (X), (X)bis, (XI), (XI)bis, (XII), (XII)bis, (XIII) et (XIII)bis:

Les composés de formule générale **(X)** et **(X)*bis*** sont obtenus à partir de la coupure d'un groupe protecteur. Les composés de formule générale **(X)** et **(X)*bis***, dans lesquels X', Y, Y', Ω, P et A sont tels que définis ci-dessus, peuvent être préparés à partir des composés de formule générale **(XI)** et **(XI)*bis*,** schéma 3, qui sont des composés comportant une amine / aniline protégée (NHGp) sous forme, par exemple, d'un carbamate ou d'un alcool ou phénol protégé par un groupe benzyle (O-benzyle) ou par d'autres groupes protecteurs (Gp₁, Gp₂) connus de l'homme du métier. Dans le cas particulier des groupes BOC, ceux-ci sont déprotégés classiquement à l'aide d'acide trifluoroacétique (TFA) ou de HCl, les groupes O-benzyle étant quant à eux déprotégés classiquement par hydrogénation catalytique en présence de Pd sur charbon, et l'on obtient finalement les composés de formule générale **(X)** et **(X)*bis***.

Les composés de formule générale **(XI)** et **(XI)*bis*** peuvent être préparés à partir des intermédiaires de formule générale **(XII)** et **(XII)*bis*** et **(XIII)** et **(XIII)*bis*** selon le schéma 3 où Ω, Y, X', Y', P sont tels que définis ci-dessus et Gp₁ et Gp₂ sont des groupes protecteurs.

Les dérivés d'aniline / d'amines de formule générale **(XII)** et **(XII)*bis*** peuvent être condensés sur des composés de formule générale **(I.i, Iii,** et **Iiii),** dans lesquels L représente un groupe partant et avec **(I.iv** et **I.v)** comme précédemment décrit pour les composés de formule générale **(I)** dans le schéma 1, pour conduire finalement aux composés de formule générale **(X)** et **(X)*bis***, schéma 3.

Les composés de formule générale **(XII)** et **(XII)***bis*, sont obtenus à partir de la réduction d'un groupement nitro des composés de formule générale **(XIII)** et **(XIII)*bis***. La réduction de la fonction nitro des composés de formule générale **(XIII)** et **(XIII)*bis***, schéma 3, dans lesquels Ω, Y, X', Y' et P sont tels que définis ci-dessus, est effectuée, par exemple, classiquement par hydrogénation catalytique en présence de Pd sur charbon, ou bien en chauffant le produit dans un solvant approprié tel que l'acétate d'éthyle avec un peu d'éthanol en présence de SnCl₂ (*J*. *Heterocyclic Chem.* (1987), 24, 927-930 ; *Tetrahedron Letters* (1984), 25 (8), 839-842), en présence de SnCl₂ / Zn (*Synthesis*. (1996), 9, 1076-1078) ou encore à l'aide de NaBH₄-BiCl₃ (*Synth*. *Com.* (1995) 25 (23), 3799-3803) dans un solvant tel que l'éthanol, ou alors en utilisant du Ni Raney additionné d'hydrate d'hydrazine (*Monatshefte für Chemie,* (1995), 126, 725-732), ou enfin à l'aide d'indium dans un mélange d'éthanol et de chlorure d'ammonium à reflux (*Synlett (1998) 9*, 1028), pour conduire finalement aux amines et anilines primaires de formule générale **(XII)** et **(XII)***bis*.

La synthèse des composés de formule générale **(XIII)** et **(XIII)*bis*** non commerciaux, est décrite plus loin.

### Préparation de certains intermédiaires de synthèse non commerciaux :

Les acides de formule générale **(I.vi)** non commerciaux, dans lesquels m est tel que défini ci-dessus sont accessibles à partir de méthodes de la littérature. Par exemple, l'acide trisnorlipoïque [acide 2-(1,2-dithiolan-3-yl)-acétique] est obtenu en 5 étapes selon un protocole expérimental décrit dans *Tetrahedron Letters*.(1997), **38** (33), 5785-5788.

Les composés de formule générale **(XIII)*bis*** dans lesquels P représente un radical phénylène, Ω représente un radical pipérazinyle ou homopipérazinyle (T représente le radical -(CH₂)ₓ- avec x = 2 ou 3) et r et R₆ sont tels que définis dans la formule générale **(I)**, schéma 3.1, sont préparés à partir des dérivés halogénés de formule générale **(XIII.1)*bis***. Ces derniers sont soumis à une substitution nucléophile par un composé hétérocyclique de formule générale **(XIII.2)*bis*** dans un solvant comme le DMSO, le DMF, l'acétone ou le dichlorométhane en présence d'une base telle que K₂CO₃, KOH, NaOH ou la triéthylamine, pour conduire aux intermédiaires de formule générale **(XIII)*bis***.

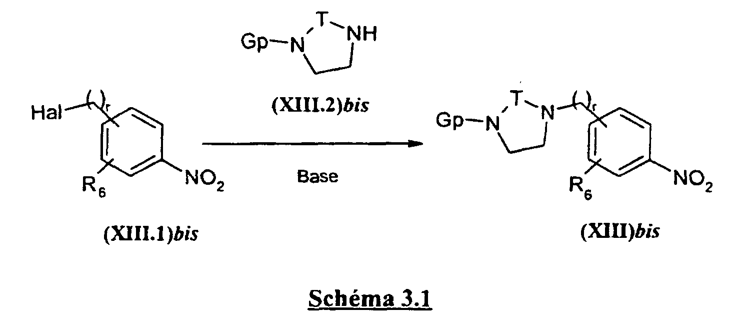

Certains composés de formule générale **(XIII)***bis* dans lesquels P représente un radical phénylène, Ω représente un radical pipéridine, homopipéridine ou pyrrolidine (T représente le radical -(CH₂)_{y}- avec y = 1, 2 ou 3) et R₆ est tel que défini dans la formule générale **(I)** peuvent être préparés, schéma 3.2, à partir des nitrobenzaldéhydes substitués de formule générale **(XIII.3)*bis***. Les composés substitués de formule générale **(XIII.3)*bis*** sont condensés avec une amine de formule générale **(XIII.4)*bis*** en milieu réducteur. La réaction a lieu dans un solvant alcoolique tel que, par exemple, le méthanol, en présence d'un agent réducteur tel que, par exemple, NaBH₄ ou NaBH₃CN. Quand R₃ = H, la protection de la fonction amine libre des composés de formule générale **(XIII)*bis***, est effectuée classiquement avec (Boc)₂ dans du dichlorométhane ou par d'autres techniques de protection connues de l'homme de métier.

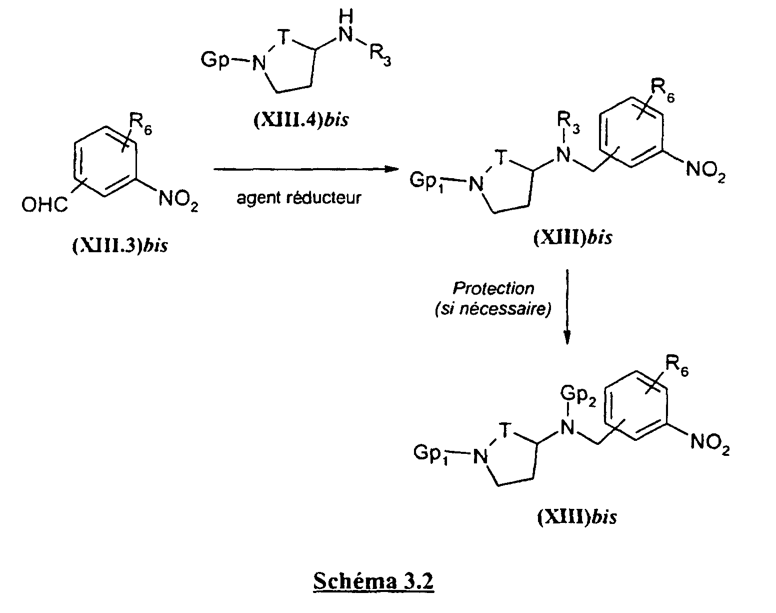

Les composés de formule générale **(XIII)*bis*** dans lesquels P représente un radical phénylène, Ω représente un radical pipérazinyle ou homopipérazinyle (T représente le radical -(CH₂)_{z}- avec z = 2 ou 3), r est tel que défini ci-dessus et R₆ est un radical aminométhyle protégé par un groupe protecteur Gp₂, schéma 3.3, sont préparés à partir des dérivés halogénés de formule générale **(XIII.5)*bis***. La réduction de la fonction nitrile des intermédiaires de formule générale **(XIII.6)*bis*** est effectuée, par exemple, dans un solvant approprié tel que l'éther ou le THF, en présence d'un agent réducteur comme par exemple le diborane. La protection de l'amine primaire formée est ensuite effectuée classiquement avec (Boc)₂ dans du dichlorométhane ou par d'autres techniques de protection connues de l'homme de métier pour conduire aux composés de formule générale **(XIII)*bis***.

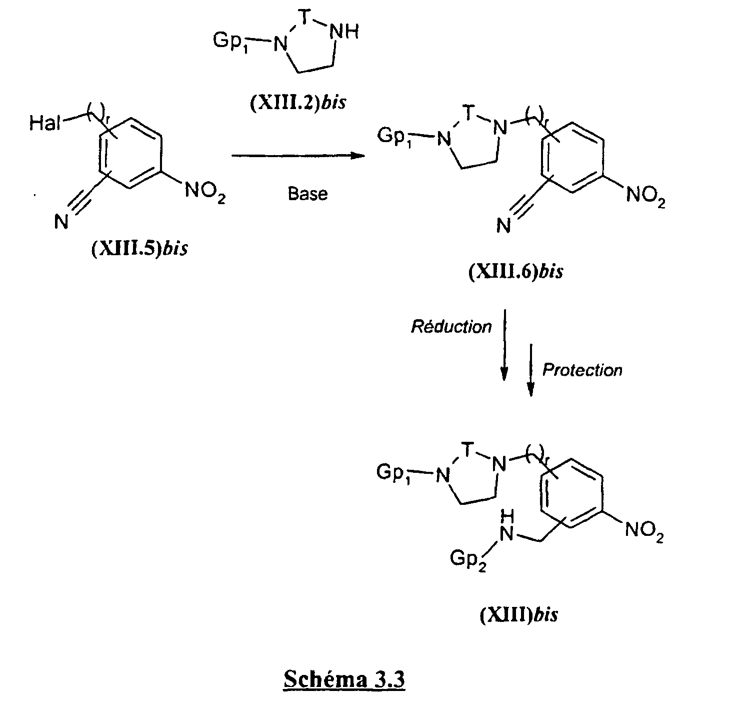

Les composés de formule générale **(XI)** dans lesquels Ω représente un radical phénylène substitué par un radical R₅ et r est tel que défini dans la formule générale **(I)**, sont préparés, schéma 3.4, par couplage peptidique (dans des conditions classiques de synthèse peptidique telles celles déjà décrites précédemment) des acides de formule générale **(XI.1)** avec les amines de formule générale **(XI.2)**. La préparation des amines de formule générale **(XI.2)** peut être effectuée, par exemple, de façon analogue au mode opératoire des étapes 17.2 à 17.6 de l'exemple 17 décrit plus loin. Le groupe R₅ est éventuellement préalablement protégé lorsqu'il comporte une fonction amine, aniline, alcool ou phénol libre, la déprotection de ladite fonction amine, aniline, alcool ou phénol libre ayant en principe lieu une fois la réaction de couplage peptidique entre le composé de formule générale **(I.vi)** et le composé de formule générale **(X)** effectuée (ces réactions, effectuées selon des méthodes courantes pour l'homme du métier, ne sont pas indiquées dans le schéma 3.4).

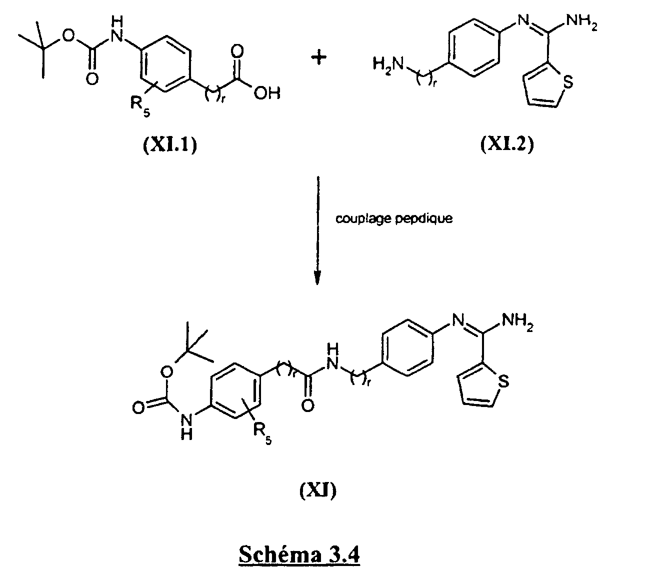

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevets, tous les brevets et toutes autres références mentionnées ici sont incorporées par référence.

Les exemples suivants sont présentés pour illustrer les procédures ci-dessus et ne doivent en aucun cas être considérés comme une limite à la portée de l'invention.

### EXEMPLES

### Exemple 1 : chlorhydrate de N'-(4-{4-[5-(1,2-dithiolan-3-yl)pentanoyl]-1-pipérazinyl}phényl)-2-thiophènecarboximidamide :

### A. Méthode selon la première approche :

### 1.A.1) 1-[5-(1,2-dithiolan-3-yl)pentanoyl]-4-(4-nitrophényl)pipérazine :

A une solution de 5,0 g (24,13 mmol) d'acide (DL)-thioctique dans 120 ml de dichlorométhane, on ajoute successivement 5,0 g (24,13 mmol) de nitrophénylpipérazine, de la triéthylamine (14,0 ml), 4,24 g (31,37 mmol) d'hydroxybenzotriazole et 12,0 g (62,73 mmol) de chlorhydrate de 1-(3-diméthylaminopropyl)-3-éthyl-carbodiimide. Après avoir agité le mélange réactionnel une nuit à 25° C, on dilue l'ensemble avec 400 ml d'eau et l'agitation est maintenue 30 minutes supplémentaires. Le produit est extrait à l'aide de 3 fois 200 ml de dichlorométhane. La solution organique est séchée sur sulfate de magnésium, filtrée et concentrée sous vide. Le solide obtenu est filtré et rincé à l'éther diéthylique pour obtenir, après séchage, 6,21 g d'un produit solide jaune avec un rendement de 65 %. Point de fusion : 97,6-98,5° C.

### 1.A.2) 4-{4-[5-(1,2-dithiolan-3-yl)pentanoyl]-1-pipérazinyl}aniline :

A une solution de 5,2 g (13,20 mmol) de l'intermédiaire 1.A.1 dans 60 ml d'éthanol, on ajoute successivement 16 ml d'une solution aqueuse de chlorure d'ammonium saturée et 21,0 g (0,183 mol) d'Indium en poudre puis l'on porte le milieu réactionnel à reflux pendant 5 heures (*Synlett* (1998) **9,** 1028). L'ensemble est refroidi à température ambiante et filtré sur célite. Le filtrat est alcalinisé à pH 10 avec une solution d'hydroxyde de sodium à 50 %. Le produit réduit est extrait à l'aide de 4 fois 150 ml de dichlorométhane. La solution organique est séchée sur sulfate de magnésium, filtrée et concentrée sous vide pour conduire à une huile jaune. On dissout l'huile dans un mélange de 15 ml de diméthylformamide et 20 ml d'acétate d'éthyle. L'ensemble est refroidi à l'aide d'un bain de glace, à 0° C, et on ajoute goutte à goutte une solution aqueuse de bicarbonate de potassium à 10 %. Après avoir agité le mélange réactionnel environ 10 minutes à 0° C, on ajoute la solution d'Iode (3,45 g dans 40 ml d'acétate d'éthyle) goutte à goutte jusqu'à ce que la coloration de l'iode persiste. Le produit est extrait à l'aide de 4 fois 100 ml de l'acétate d'éthyle, la solution organique est séchée sur sulfate de magnésium, filtrée et concentrée sous vide. On procède alors à une purification sur colonne de silice (éluant = 5 % d'éthanol dans dichlorométhane) pour conduire à une huile jaune, 3,03 g, avec un rendement de 63 %.
MH⁺ = 366,2.
RMN ¹H (DMSO d6, 400 MHz, δ) : 1,39 (m, 2H, CH₂) ; 1,50-1,70 (m, 4H, CH₂) ; 1,87 (m, 1H, CH₂) ; 2,32 (m, 2H, CH₂) ; 2,34 (m, 1H, CH₂); 2,80-2,88 (m, 4H, CH₂-pipérazine) ; 3,17 (m, 2H, CH₂); 3,53 (m, 4H, CH₂-pipérazine); 3,59 (m, 1H, -S-CH-); 4,58 (s, 2H, NH₂); 6,50 (d, 2H, arom., J = 7,0 Hz) ; 6,69 (d, 2H, arom., J = 7,0 Hz).

### 1.A.3) Chlorhydrate de N'-(4-{4-[5-(1,2-dithiolan-3-yl)pentanoyl]-1-pipérazinyl}phényl)-2-thiophènecarboximidamide :

L'intermédiaire 1.A.2 (0,5 g ; 1,36 mmol) est dissous dans du 2-propanol (15 ml) et on ajoute 0,582 g d'iodhydrate de S-méthyl-2-thiophène thiocarboximide (2,04 mmol) (*Ann*. *Chim*., (1962), **7**, 303-337). Après agitation à 25° C pendant 15 heures, le mélange réactionnel est concentré à sec sous vide. Le résidu est repris dans du dichlorométhane et une solution aqueuse saturée de NaHCO₃. Après décantation, la phase organique est lavée successivement avec 50 ml d'une solution saturée de NaHCO₃, d'eau puis de saumure. La solution organique est séchée sur sulfate de magnésium, filtrée et évaporée sous pression réduite. On dissout ensuite la base libre dans 5 ml d'éthanol et la solution est refroidie à l'aide d'un bain de glace avant l'addition goutte à goutte de 0,3 ml d'une solution 1N de HCl dans de l'éther éthylique anhydre. Après 15 heures d'agitation à 25° C, les cristaux obtenus sont filtrés et rincés à l'éther diéthylique pour obtenir après séchage 0,320 g d'un produit solide jaune avec un rendement de 43 %. Point de fusion : 203-203,7° C.

### B. Méthode selon la deuxième approche :

### 1.B.1) 4-(4-nitrophényl)-1-pipérazinecarboxylate de tert-butyle

On dissout 5.55 g (26.5 mmol) de 1-(4-nitrophényl)pipérazine dans un mélange de 60 ml de dichlorométhane et 8.2 ml de triéthylamine. L'ensemble est refroidi à l'aide d'un bain de glace avant l'addition en plusieurs portions de 6,4 g (29,2 mmol) de (Boc)₂O. Le mélange réactionnel est agité à 23° C pendant 12 heures et versé dans un mélange eau-glace. La phase organique est décantée, lavée successivement par 20 ml d'eau et 20 ml de saumure. Après séchage sur sulfate de sodium, filtration et concentration sous vide, puis trituration avec de l'éther isopropylique, on obtient un solide jaune avec un rendement quantitatif. Point de fusion : 143,7-145,7° C.

### 1.B.2) 4-(4-aminophényl)-1-pipérazinecarboxylate de tert-butyle

Dans un autoclave en acier inox équipé d'un barreau magnétique, on introduit une solution de l'intermédiaire 1.B.1 (7,63 g ; 25,0 mmol) dans 40 ml d'un mélange dichlorométhane/éthanol (30 ml / 50 ml) ainsi que 1,0 g de Pd/C à 10 %. Le mélange réactionnel est agité sous pression d'hydrogène (1,5 bar) pendant 12 heures à une température de 20° C. Le Pd/C est ensuite éliminé par filtration et le filtrat est concentré sous vide. Le résidu d'évaporation est purifié par trituration avec l'éther, on obtient une poudre grise (4,96 g ; rendement de 71 %). Point de fusion : 145° C.

### 1.B.3) 4-(4-{[amino(2-thienyl)méthylidène]amino}phényl)-1-pipérazinecarboxylate de tert-butyle

L'intermédiaire 1.B.2 (4.96 g ; 17,9 mmol) est dissous dans du 2-propanol (150 ml) et l'on ajoute 7,66 g d'iodhydrate de S-méthyl-2-thiophène thiocarboximide (26,8 mmol) (Ann. Chim., (1962), **7**, 303-337). Après agitation à 25° C pendant 15 heures, le mélange réactionnel est concentré à sec sous vide. Le résidu est repris dans du dichlorométhane et une solution aqueuse saturée de NaHCO₃. Après décantation, la phase organique est lavée successivement par 50 ml d'une solution saturée de NaHCO₃, d'eau et de saumure. La solution organique est séchée sur sulfate de magnésium, filtrée et évaporée sous pression réduite. Le solide obtenu est filtré et rincé à l'isopentane pour obtenir, après séchage, 6,91 g d'un produit solide jaune (rendement de 78 %). Point de fusion : 164°C

### 1.B.4) N'-[4-(1-pipérazinyl)phényl]-2-thiophènecarboximidamide :

Dans une solution à 0° C de l'intermédiaire 1.B.3 (5,40 g ; 14,0 mmol) dans de l'acétate d'éthyle (200 ml), on passe un courant d'HCl gaz bulle à bulle. On laisse le mélange revenir à température ambiante durant la nuit. On fait passer un courant d'Argon à travers la masse réactionnelle, puis la poudre obtenue est filtrée et lavée avec l'acétate d'éthyle pour conduire à un solide blanc-beige (5,0 g ; rendement de 99 %). Point de fusion: 180° C.

### 1.B.5) Chlorhydrate de N'-(4-{4-[5-(1,2-dithiolan-3-yl)pentanoyl]-1-pipérazinyl}phényl)-2-thiophènecarboximidamide :

Le protocole expérimental utilisé est analogue à celui décrit pour le composé 1.A.1. Le chlorhydrate est fait de la même façon que pour l'intermédiaire 1.A.3. On obtient le solide jaune attendu.

### Exemple 2 : N'-(4-{4-[2-(1,2-dithiolan-3-yl)acétyl]-1-pipérazinyl}phényl)-2-thiophènecarboximidamide :

Le protocole expérimental utilisé est le même que celui décrit pour le composé 1, méthode A. L'acide norlipoïque, (*Tetrahedron Letters* (1997), **38** (33), 5785-5788), remplaçant l'acide lipoïque, pour conduire à un solide jaune (0,126 g).
MH⁺= 433,2.
RMN ¹H (DMSO d6, 400 MHz, δ) : 1,97 (m, 1H, CH₂) ; 2,40 (m, 1H, CH₂) ; 2,75 (m, 1H,CH₂); 2,87 (m, 1H,CH₂); 3.04 (m, 4H, CH₂-pipérazine); 3,09 (m, 1H,CH₂) ; 3,21 (m, 1H, CH₂); 3,59 (m, 4H, CH₂-pipérazine); 3,97 (m, 1H, -S-CH-); 6,26 (s large, 2H, NH₂) ; 6,76 (d, 2H, arom., J = 8,6 Hz) ; 6,94 (d, 2H, arom., J = 8,6 Hz) ; 7,07 (m, 1H,CH-thiophène) ; 7,57 (m, 1H,CH-thiophène) ; 7,70 (m, 1H,CH-thiophène).

### Exemple 3 : chlorhydrate de N'-[3-({4-[5-(1,2-dithiolan-3-yl)pentanoyl]-1-pipérazinyl}méthyl)phényl]-2-thiophènecarboximidamide :

Le protocole expérimental utilisé est le même que celui décrit pour le composé 1, méthode A. On obtient un solide blanc (0,220 g). Point de fusion : 240,0-240,5° C.

### Exemple 4 : N'-[3-({4-[2-(1,2-dithiolan-3-yl)acétyl]-1-pipérazinyl}méthyl)phényl]-2-thiophènecarboximidamide :

Le protocole expérimental utilisé est le même que celui décrit pour le composé 1, méthode A. L'acide norlipoïque, (*Tetrahedron Letters* (1997), **38** (33), 5785-5788), remplaçant l'acide lipoïque, pour conduire à une mousse jaune (0,20 g).
MH⁺ = 447,2.
RMN ¹H (DMSO d6, 400 MHz, δ) : 1,96 (m, 1H, CH₂); 2,34-2,40 (m, 4H, CH₂-pipérazine) ; 2,49 (m, 1H, CH₂) ; 2,71 (m, 1H,CH₂) ; 2,80 (m, 1H, CH₂); 3,08 (m, 1H, CH₂) ; 3,28 (m, 1H, CH₂) ; 3,41 (m, 4H, CH₂-pipérazine) ; 3,46 (3, 2H, CH₂) ; 3,93 (m, 1H, -S-CH-); 6,54 (s large, 2H, NH₂) ; 6,77 (d, 1H, arom., J = 7,60 Hz) ; 6,82 (s, 1H, arom); 6,95 (d, 1H, arom., J = 7,60 Hz) ; 7,10 (m, 1H,CH-thiophène); 7,26 (t, 1H, arom., J = 7,60 Hz); 7,63 (m, 1H, CH-thiophène); 7,74 (m, 1H, CH-thiophène).

### Exemple 5 : 4-(4-{[amino(2-thiényl)méthylidène}amino}phényl)-N-[4-(1,2-dithiolan-3-yl)butyl]-1-pipérazinecarboxamide :

Sous atmosphère d'argon, on solubilise 0,206 g (1,0 mmol) d'acide lipoïque dans du dioxane (5 ml), de l'acétonitrile (5 ml) et de la triéthylamine (0,3 ml). Du diphénylphosphoryl azide (0,24 ml ; 1,0 mmol) est ajouté puis le milieu réactionnel porté à 80° C pendant 2 heures. Le milieu réactionnel est alors refroidi à l'aide d'un bain de glace puis l'intermédiaire 1.B.4 (0,395 g; 1,0 mmol) additionné. Après avoir agité le mélange réactionnel une nuit à 25° C, on dilue l'ensemble avec 100 ml d'eau et l'agitation est maintenue 30 minutes supplémentaires. Le produit est extrait à l'aide de 3 fois 25 ml d'acétate d'éthyle. La solution organique est séchée sur sulfate de magnésium, filtrée et concentrée sous vide. On procède alors à une purification sur colonne de silice (éluant = 10 % d'éthanol dans du dichlorométhane) pour conduire à un solide jaune. Le solide jaune est purifié davantage par recristallisation dans de l'éthanol pour donner 0,25 g de produit (rendement de 46,4 %). Point de fusion : 201,1-201,8° C.

### Exemple 6 : 4-(4-{[amino(2-thiényl)méthylidene]amino}-2-méthylphényl)-N-[4-(1,2-dithiolan-3-yl)butyl]-1-pipérazinecarboxamide

### 6.1. 4-(2-méthyl-4-nitrophényl)-1-pipérazinecarboxylate de tert-butyle

Sous atmosphère d'argon, on solubilise 4,65 g (3,10 mmol) de 2-fluoro-5-nitrotoluène, 6,71 g (3,60 mmol) de 1-pipérazinecarboxylate de *tert*-butyle et 10,4 g (7,50 mmol) de carbonate de potassium dans du DMF sec (50 ml), puis le milieu réactionnel est porté à 80° C pendant 18 heures. Le milieu réactionnel est alors refroidi à l'aide d'un bain de glace puis versé sur de l'eau glacée. Le produit est extrait à l'aide de 3 fois 50 ml d'acétate d'éthyle. La solution organique est séchée sur sulfate de magnésium, filtrée et concentrée sous vide. On procède alors à une purification par cristallisation dans de l'éther diisopropylique et le solide obtenu est filtré et rincé à l'isopentane pour obtenir, après séchage. 5,9 g d'un produit solide jaune (rendement de 62 %). Point de fusion : 121,8-123,4° C.

### 6.2. 4-(4-amino-2-méthylphényl)-1-pipérazinecarboxylate de tert-butyle

Le protocole expérimental utilisé est le même que celui décrit pour le composé 1.B.2., l'intermédiaire 6.1 remplaçant l'intermédiaire 1.B.1 et l'éthanol remplaçant le mélange dichlorométhane/éthanol pour conduire à une mousse beige qui est suffisamment pure pour être utilisée dans l'étape suivante (rendement de 98 %).
MH⁺ = 292,20.

### 6.3. 4-(4-{[amino(2-thiényl)méthylidène]amino}-2-méthylphényl)-1-pipérazinecarboxylate de tert-butyle

Le protocole expérimental utilisé est le même que celui décrit pour le composé 1.B.3., l'intermédiaire 6.2 remplaçant l'intermédiaire 1.B.2. Après purification par cristallisation dans l'éther, le solide obtenu est filtré et rincé à l'éther diisopropylique, pour obtenir, après séchage, un produit solide blanc-jaune pâle (rendement de 71 %). Point de fusion : 165° C.

### 6.4. Chlorhydrate de N'-[3-méthyl-4-(1-pipérazinyl)phényl]-2-thiophènecarboximidamide

Le protocole expérimental utilisé est le même que celui décrit pour le composé 1.B.4., l'intermédiaire 6.3 remplaçant l'intermédiaire 1.B.3.. On obtient un produit solide-mousse de couleur blanche (rendement de 95 %).
MH⁺ = 301,20.

### 6.5. 4-(4-{[-amino(2-thiényl)méthylidène]amino}-2-méthylphényl)-N-[4-(1,2-dithiolan-3-yl)butyl]-1-pipérazinecarboxamide

Le protocole expérimental utilisé est le même que celui décrit pour le composé de l'exemple 5, l'intermédiaire 6.4 remplaçant l'intermédiaire 1.B.4. On obtient un produit solide blanc-jaunâtre (rendement de 3 %). Point de fusion : 193,3-194,6° C.

### Exemple 7 : 4-(4-{[amino(2-thiényl)méthylidène]amino}-3-méthylphényl)-N-[4-(1,2-dithiolan-3-yl)butyl]-1-pipérazinecarboxamide

Le protocole expérimental utilisé est le même que celui décrit pour le composé de l'exemple 6, le 5-fluoro-2-nitrotoluène remplaçant le 2-fluoro-5-nitrotoluène. On obtient un produit solide blanc (rendement de 16 %). Point de fusion : 169,4-170,9° C.

### Exemple 8 : 4-(4-{[amino(2-thiényl)méthylidène]amino}-2-méthoxyphényl)-N-[4-(1,2-dithiolan-3-yl)butyl]-1-pipérazinecarboxamide

Le protocole expérimental utilisé est le même que celui décrit pour le composé de l'exemple 6, le 2-chloro-5-nitroanisole remplaçant le 2-fluoro-5-nitrotoluène. On obtient un produit solide jaune pâle (rendement de 25 %). Point de fusion : 185,6-186,3° C.

### Exemple 9 : 4-(4-{[amino(2-thiényl)méthylidène]amino}-3-méthoxyphényl)-N-[4-(1,2-dithiolan-3-yl)butyl]-1-pipérazinecarboxamide

Le protocole expérimental utilisé est le même que celui décrit pour le composé de l'exemple 6, le 5-chloro-2-nitroanisole remplaçant le 2-fluoro-5-nitrotoluène. On obtient une mousse jaune pâle (rendement de 4 %).
MH⁺ = 520,20.

### Exemple 10 : 4-(4-{[amino(2-thiényl)méthylidène]amino}-2-cyanophényl)-N-[4-(1,2-dithiolan-3-yl)butyl]-1-pipérazinecarboxamide

Le protocole expérimental utilisé est le même que celui décrit pour le composé de l'exemple 6, le 2-fluoro-5-nitrobenzonitrile remplaçant le 2-fluoro-5-nitrotoluène. On obtient un solide jaune (rendement de 12 %).
MH⁺ = 515,30.

### Exemple 11 : 4-(3-{[amino(2-thiényl)méthylidène]amino}benzyl)-N-[4-(1,2-dithiolan-3-yl)butyl]-1-pipérazinecarboxamide

### 11.1. 4-(3-nitrobenzyl)-1-pipérazinecarboxylate de tert-butyle

Sous atmosphère d'argon, on solubilise 5,00 g (2,30 mmol) de 3-nitrobromobenzyle et 4,53 g (2,40 mmol) de 1-pipérazinecarboxylate de *tert*-butyle dans du dichlorométhane (100 ml), puis on ajoute goutte à goutte de la triéthylamine (8 ml). Le milieu réactionnel est laissé agité une nuit à température ambiante avant d'être versé sur de l'eau glacée. Le produit est extrait à l'aide de 3 fois 150 ml de dichlorométhane. La solution organique est séchée sur sulfate de magnésium, filtrée et concentrée sous vide. On procède alors à une purification sur colonne de silice (éluant = gradient allant de l'heptane pur à l'acétate d'éthyle pur) pour conduire à une huile jaune. Après cristallisation à froid, on obtient 6,2 g d'un solide jaune (rendement de 84 %). Point de fusion : 85,5° C.

### 11.2. 4-(3-aminobenzyl)-1-pipérazinecarboxylate de tert-butyle

Le protocole expérimental utilisé est le même que celui décrit pour le composé 1.A.2, l'intermédiaire 11.1 remplaçant l'intermédiaire 1.A.1. On obtient une huile jaune qui, après cristallisation à froid, donne un solide jaune (rendement de 98 %). Point de fusion : 102,3 °C.

### 11.3. Iodhydrate de 4-(3-{[amino(2-thienyl)méthylidène]amino}benzyl)-1-pipérazinecarboxylate de tert-butyle

Le protocole expérimental utilisé est le même que celui décrit pour le composé 1.B.3, l'intermédiaire 11.2 remplaçant l'intermédiaire 1.B.2. On obtient un solide blanc. Point de fusion: 161,4 °C.

### 11.4. N'-[3-(1-pipérazinylméthyl)phényl]-2-thiophènecarboximidamide

Le protocole expérimental utilisé est le même que celui décrit pour le composé 1.B.4, l'intermédiaire 11.3 remplaçant l'intermédiaire 1.B.3. On obtient un solide jaune hygroscopique (rendement de 72%).
MH⁺ = 301,10.

### 11.5. 4-(3-{[amino(2-thienyl)méthylidène]amino}benzyl)-N-[4-(1.2-dithiolan-3-yl)butyl]-1-pipérazinecarboxamide

Le protocole expérimental utilisé est le même que celui décrit pour le composé de l'exemple 5, l'intermédiaire 11.4 remplaçant l'intermédiaire 1.B.4. On obtient un solide jaune pâle (rendement de 2%). Point de fusion : 152,0-153,4 °C.

### Exemple 12: N'-(3-{4-[5-(1,2-dithiolan-3-yl)pentanoyl]-1-pipérazinyl}phényl)-2-thiophènecarboximidamide

### 12.1. 4-(3-nitrophényl)-1-pipérazinecarboxylate de tert-butyle

Sous atmosphère d'argon, on solubilise 5,0 g (3,54 mmol) de 1-fluoro-3-nitrotoluène, 9,89 g (5,31 mmol) de 1-pipérazinecarboxylate de *tert*-butyle et 27,0 g (19,5 mmol) de carbonate de potassium dans du DMSO (60 ml), puis le milieu réactionnel est porté à 100 °C pendant 72 heures. Le milieu réactionnel est ensuite refroidi à l'aide d'un bain de glace puis versé sur de l'eau glacée. Le produit est extrait à l'aide de 3 fois 50 ml de dichlorométhane. La solution organique est séchée sur sulfate de magnésium, filtrée et concentrée sous vide. On procède alors à une purification sur colonne de silice (éluant = heptane 70% / acétate d'éthyle 30%) pour conduire à un solide jaune. Le solide jaune est purifié davantage par recristallisation dans de l'éther diisopropylique pour donner 4,5 g du produit attendu (rendement de 42,0%). Point de fusion : 82,0-92,0 °C.

### 12.2. 4-(3-aminophényl)-1-pipérazinecarboxylate de tert-butyle

Le protocole expérimental utilisé est le même que celui décrit pour le composé 1.B.2, l'intermédiaire 13.1 remplaçant l'intermédiaire 1.B.1 et un mélange dichlorométhane/THF (50/50) remplaçant le mélange dichlorométhane/éthanol. Après purification par cristallisation dans de l'éther diisopropylique, on obtient un solide blanc (rendement de 25%). Point de fusion : 218,0 °C.

### 12.3. 4-(3-{[amino(2-thiényl)méthylidène]amino}phenyl)-1-pipérazinecarboxylate de tert-butyle

Le protocole expérimental utilisé est le même que celui décrit pour le composé 1.B.3, l'intermédiaire 13.2 remplaçant l'intermédiaire 1.B.2. On obtient un solide jaune pâle (rendement de 58%). Point de fusion : 150,0 °C.

### 12.4.) Chlorhydrate de N'-[3-(1-pipérazinyl)phényl]-2-thiophènecarboximidamide

Le protocole expérimental utilisé est le même que celui décrit pour le composé 1.B.4, l'intermédiaire 13.2 remplaçant l'intermédiaire 1.B.3. On obtient un solide de couleur jaune-beige (rendement de 88%). Point de fusion : 141,4 °C.

### 12.5. N'-(3-{4-[5-(1,2-dithiolan-3-yl)pentanoyl]-1-pipérazinyl}phényl)-2-thiophenecarboximidamide

Le protocole expérimental utilisé est analogue à celui décrit pour le composé 1.A.1, l'intermédiaire 13.3 remplaçant la nitrophénylpiperazine. On obtient le produit attendu sous forme de mousse jaune clair (rendement de 63%).
MH⁺ = 475,20.

### Exemple 13: 5-{[amino(2-thiényl)méthylidène]amino}-2-{4-[5-(1,2-dithiolan-3-yl)pentanoyl]-1-pipérazinyl}benzylcarbamate de tert-butyle

### 13.1. 2-(4-benzyl-1-pipérazinyl)-5-nitrobenzonitrile

Le protocole expérimental utilisé est le même que celui décrit pour le composé 6.1, le 2-fluoro-5-nitrobenzonitrile remplaçant le 2-fluoro-5-nitrotoluène et la 1-benzylpipérazine remplaçant le 1-piperazinecarboxylate de *tert*-butyle. On obtient un solide de couleur ocre clair (rendement de 89%). Point de fusion : 124,0 °C.

### 13.2. [2-(4-benzyl-1-pipérazinyl)-5-nitrophényl]méthanamine

Sous atmosphère d'argon, on solubilise 3,90 g (1,21 mmol) de l'intermédiaire 14.1 dans du THF sec (40 ml) et on ajoute goutte à goutte une solution de diborane (1M dans THF ; 24,2 ml ; 2,42 mmol ) avant de porter le mélange résultant au reflux pendant 18 heures. Du méthanol (5 ml) est ajouté puis on laisse agiter. HCl gazeux est ensuite laissé barboter dans le milieu réactionnel. Après évaporation à sec du mélange, on dilue le résidu avec de l'eau (50 ml), on rend la solution alcaline avec du bicarbonate de sodium (10%) et on extrait au dichlorométhane (3 fois 100 ml). La solution organique est ensuite séchée sur sulfate de magnésium, filtrée et concentrée sous vide. On procède alors à une purification sur colonne de silice (éluant = dichlorométhane contenant 5% d'éthanol) pour conduire à une huile brune (rendement de 42,0%).
MH+ = 327,20.

### 13.3. 2-(4-benzyl-1-pipérazinyl)-5-nitrobenzylcarbamate de tert-butyle

Le protocole expérimental utilisé est le même que celui décrit pour le composé 1.B.1, l'intermédiaire 14.2 remplaçant la 1-(4-nitrophényl)pipérazine et la diisopropyléthylamine remplaçant la triéthylamine. Après purification sur colonne de silice (éluant = dichlorométhane contenant 5% d'éthanol) pour conduire à une huile jaune (rendement de 91,0%).
MH+ = 427,20.

### 13.4. 5-amino-2-(1-pipérazinyl)benzylcarbamate de tert-butyle

Le protocole expérimental utilisé est le même que celui décrit pour le composé 1.B.2, l'intermédiaire 14.3 remplaçant l'intermédiaire 1.B.1 et un mélange dichlorométhane/THF (50/50) remplaçant le mélange dichlorométhane/éthanol. On obtient une mousse blanche qui est suffisamment pure pour être utilisée dans l'étape suivante (rendement de 95%).
MH+ = 307,20.

### 13.5. 5-{[amino(2-thiényl)méthylidène]amino}-2-(1-pipérazinyl)benzylcarbamate de tert-butyle

Le protocole expérimental utilisé est le même que celui décrit pour le composé 1.B.3, l'intermédiaire 14.4 remplaçant l'intermédiaire 1.B.2 tandis que l'on ajoute dans le milieu réactionnel deux équivalents de pyridine sèche. On obtient après traitement une mousse jaune qui est suffisamment pure pour être utilisée dans l'étape suivante (rendement de 49%).
MH+ = 416,20.

### 13.6. 5-{[amino(2-thiényl)méthylidène]amino}-2-{4-[5-(1,2-dithiolan-3-yl)pentanoyl]-1-pipérazinyl}benzylcarbamate de tert-butyle

Le protocole expérimental utilisé est analogue à celui décrit pour le composé 1.A.1, l'intermédiaire 13.5 remplaçant la nitrophénylpipérazine. On obtient le produit attendu sous forme d'un solide blanc (rendement de 45%). Point de fusion : 162.4-164.0 °C.

### Exemple 14 : N'-(3-(aminométhyl)-4-{4-[5-(1,2-dithiolan-3-yl)pentanoyl]-1-pipérazinyl}phényl)-2-thiophènecarboximidamide

Le protocole expérimental utilisé est le même que celui décrit pour le composé 1.B.4, le composé 13.6 remplaçant l'intermédiaire 1.B.3. On obtient un produit solide jaune (rendement de 14%). Point de fusion : colle à partir de 106 °C.

### Exemple 15 : 3-{[amino(2-thiényl)méthylidène]amino}benzyl{1-[5-(1,2-dithiolan-3-yl)pentanoyl]-4-pipéridinyl}carbamate de tert-butyle

### 15.1. N-(1-benzyl-4-pipéridinyl)-N-(3-nitrobenzyl)amine

A un ballon contenant 100 ml de méthanol anhydre, sous atmosphère inerte, on ajoute successivement 5,04 g (3,33 mmol) de 3-nitrobenzaldéhyde et 7,48 ml (3,67 mmol) de 4-amino-1-benzylpipéridine. Le mélange réactionnel est agité pour faire l'imine pendant 18 heures puis on procède à l'addition, par portions, de 1,26 g (3,33 mmol) de NaBH₄. L'agitation est maintenue 24 heures supplémentaires avant addition de 5 ml d'eau glacée. Le mélange réactionnel est alors extrait par 2 fois 100 ml de CH₂Cl₂. La phase organique est lavée successivement avec 50 ml d'eau puis 50 ml de saumure, séchée sur sulfate de magnésium, filtrée et concentrée sous vide. Le résidu est purifié sur une colonne de silice (éluant : dichlorométhane contenant 5% d'éthanol). On obtient une huile jaune avec un rendement de 85%.
MH+ = 316,20.

### 15.2. 1-benzyl-4-piperidinyl(3-nitrobenzyl)carbamate de tert-butyle

Le protocole expérimental utilisé est le même que celui décrit pour le composé 1.B.1, l'intermédiaire 15.1 remplaçant la 1-(4-nitrophényl)pipérazine. On obtient, après traitement, une huile jaune, qui est suffisamment pure pour être utilisée dans l'étape suivante (rendement de 99%).
MH+ = 426,30.

### 15.3. 3-aminobenzyl(4-pipéridinyl)carbamate de tert-butyle

Le protocole expérimental utilisé est le même que celui décrit pour le composé 1.B.2, l'intermédiaire 15.2 remplaçant l'intermédiaire 1.B.1 et un mélange dichlorométhane/THF (50/50) remplaçant le mélange dichlorométhane/ethanol. On obtient à une mousse blanc-gris qui est suffisamment pure pour être utilisée dans l'étape suivante (rendement de 63%).
MH+ = 306,30.

### 15.4. 3-{[amino(2-thiényl)méthylidène]amino}benzyl(4-pipéridinyl)carbamate de tert-butyle

Le protocole expérimental utilisé est le même que celui décrit pour le composé 1.B.3, l'intermédiaire 16.3 remplaçant l'intermédiaire 1.B.2 tandis que l'on ajoute dans le milieu réactionnel deux équivalents de pyridine sèche. On obtient, après purification sur une colonne de silice (éluant : gradient allant du dichlorométhane pur à du dichlorométhane contenant 10% d'éthanol contenant des traces d'ammoniaque), une mousse blanc-jaunâtre (rendement de 62%).
MH+ = 415,20.

### 15.5. 3-{[-amino(2-thiényl)méthylidène]amino}benzyl{1-[5-(1,2-dithiolan-3-yl)pentanoyl]-4-pipéridinyl}carbamate de tert-butyle

Le protocole expérimental utilisé est analogue à celui décrit pour le composé 1.A.1, l'intermédiaire 16.4 remplaçant la nitrophénylpipérazine. On obtient le produit attendu sous forme d'une mousse jaunâtre (rendement de 28%).
MH+ = 603,20.

### Exemple 16: N'-{3-[({1-[5-(1,2-dithiolan-3-yl)pentanoyl]-4-pipéridinyl}amino)méthyl]phényl}-2-thiophènecarboximidamide

Le protocole expérimental utilisé est le même que celui décrit pour le composé 1.B.4, le composé 15.5 remplaçant l'intermédiaire 1.B.3. On obtient un solide blanc (rendement de 33%).
MH+ = 503,31.

### Exemple 17: N-[2-(4-{[amino(2-thiényl)méthylidène]amino}phényl)éthyl]-4-{[5-(1,2-dithiolan-3-yl)pentanoyl]amino}benzamide

### 17.1. Acide 4-[(tert-butoxycarbonyl)amino]benzoïque

Le protocole expérimental utilisé est le même que celui décrit pour le composé 1.B.1, l'acide 4-aminobenzoïque remplaçant la 1-(4-nitrophényl)pipérazine. On obtient un solide qui est suffisamment pur pour être utilisé dans l'étape suivante (rendement de 23%).
MH+ = 238.

### 17.2. 3,5-diméthoxy-2-({[2-(4-nitrophényl)éthyl]amino}méthyl)phénol

Dans un ballon contenant 200 ml de méthanol anhydre, sous atmosphère inerte, on ajoute successivement 9,0 g (49,4 mmol) de 4,6-diméthoxysalicaldéhyde, 11,6 g (54,3 mmol) de chlorhydrate de 4-nitrophénéthylamine et 7,5 ml de triéthylamine. Le mélange réactionnel est agité vigoureusement pendant 15 heures avant l'addition, par portions, de 2,1 g (55,5 mmol) de NaBH₄. L'agitation est maintenue 10 heures supplémentaires avant addition de 10 ml d'eau. Après un quart d'heure, le mélange réactionnel est extrait avec 2 fois 100 ml de CH₂Cl₂. La phase organique est lavée successivement avec 50 ml d'eau et 50 ml de saumure, séchée sur sulfate de sodium, filtrée et concentrée sous vide. Le résidu est alors purifié sur une colonne de silice (éluant : CH₂Cl₂/EtOH : 20/1). On obtient une huile orange avec un rendement de 58%.

### 17.3. 2-hydroxy-4,6-diméthoxybenzyl[2-(4-nitrophényl)éthyl]carbamate de tert-butyle

On dissout 30,0 mmol d'intermédiaire 17.2 dans un mélange de 100 ml de dichlorométhane et 9,2 ml de triéthylamine. L'ensemble est refroidi à l'aide d'un bain de glace avant l'addition en plusieurs portions de 7,2 g (33,0 mmol) de (Boc)₂O. Le mélange réactionnel est agité à 23 °C pendant 12 heures et versé dans un mélange eau-glace. La phase organique est décantée, lavée successivement avec 20 ml d'eau et 20 ml de saumure. Après séchage sur sulfate de sodium, filtration et concentration sous vide, on obtient après trituration avec de l'éther isopropylique un solide blanc avec un rendement de 60%. Point de fusion : 133,5-134,4 °C.

### 17.4. 2-(4-aminophényl)éthyl(2-hydroxy-4,6-diméthoxybenzyl)carbamate de tert-butyle :

Dans un autoclave en acier inox équipé d'un barreau magnétique, on introduit une solution de l'intermédiaire 17.3 (20,2 mmol) dans 66 ml d'un mélange dichlorométhane, d'acétate d'éthyle et de THF (1 ml / 60 ml / 5 ml) ainsi que 1,0 g de Pd/C à 10%. Le mélange réactionnel est agité sous pression d'hydrogène (1,5 bar) pendant 12 heures à une température de 20 °C. Le Pd/C est ensuite éliminé par filtration et le filtrat est concentré sous vide. On obtient une huile jaune clair avec un rendement de 90%.

### 17.5. 2-(4-{[(amino(2-thiényl)méthylidène]amino}phényl)éthylcarbamate de tert-butyle :

L'intermédiaire 17.4 (2,37 mmol) est dissous dans du 2-propanol (15 ml) et l'on ajoute 1,014 g d'iodhydrate de S-méthyl-2-thiophène thiocarboximide (3,56 mmol) (*Ann. Chim*. (1962), **7**, 303-337). Après chauffage à 60 °C pendant 15 heures, le mélange réactionnel est concentré à sec sous vide. Le résidu est repris dans du dichlorométhane et une solution aqueuse saturée de NaHCO₃. Après décantation, la phase organique est lavée successivement par 50 ml d'une solution saturée de NaHCO₃, d'eau et de saumure. La solution organique est séchée sur sulfate de magnésium, filtrée et évaporée sous pression réduite. On dissout ensuite la base libre dans 30 ml de dichlorométhane et la solution est refroidie à l'aide d'un bain de glace avant l'addition goutte à goutte de 6,3 ml d'une solution 1N HCl dans l'éther éthylique anhydre. Après 15 heures d'agitation à 25 °C, les cristaux obtenus sont filtrés et rincés à l'éther diéthylique pour obtenir après séchage un solide jaune avec un rendement de 79%. Point de fusion : 144 °C.

### 17.6. N'-[4-(2-aminoéthyl)phényl]-2-thiophènecarboximidamide :

Dans une solution de l'intermédiaire 17.5 (16,4 mmol) dans un mélange (200 ml) d'éther/éthanol/acétone/dichlorométhane (1/1/1/1), on fait passer un courant d'HCl gaz bulle à bulle à 0 °C. On laisse remonter à température ambiante toute la nuit. On fait passer un courant d'argon à travers la masse réactionnelle et on évapore les solvants à sec. Le résidu d'évaporation est ensuite versé dans 100 ml d'une solution saturée de NaHCO₃ froide et extrait par 3 fois 100 ml de dichlorométhane. La phase organique est séchée sur sulfate de magnésium, filtrée et concentrée sous vide. On procède alors à une purification sur colonne de silice (éluant = heptane à 50 % acétate d'éthyle puis dichlorométhane à 5 % éthanol) pour conduire à un solide blanc avec un rendement de 79%. Point de fusion : 169,2-170,5°C.

### 17.7. 4-amino-N-[2-(4-{[amino(2-thiényl)méthylidène]amino}phényl)éthyl]-benzamide

Le protocole expérimental utilisé est analogue à celui décrit pour le composé 1.A.1, l'intermédiaire 17.1 remplaçant l'acide (DL)-thioctique et l'intermédiaire 17.6 remplaçant la nitrophénylpipérazine. On obtient le produit attendu sous forme d'une mousse blanche avec un rendement de 35% (ainsi qu'un produit secondaire, le 4-({[2-(4- {[amino(2-thiényl)méthylidène]amino}phényl)éthyl]amino}carbonyl)phénylcarbamate de tert-butyle, sous forme d'un solide blanc, avec un rendement de 14%).
MH+ = 365,20.

### 17.8. N-[2-(4-{[amino(2-thiényl)méthylidène]amino}phényl)éthyl]-4-{[5-(1,2-dithiolan-3-yl)pentanoyl]amino}benzamide

Le protocole expérimental utilisé est analogue à celui décrit pour le composé 1.A.1, l'intermédiaire 17.7 remplaçant la nitrophénylpipérazine. On obtient le produit attendu sous forme de cristaux jaune pâle (rendement de 17%).
MH+ = 553,10.

### Etude pharmacologique des produits de l'invention :

### Etude des effets sur la NO synthase constitutive neuronale de cervelet de rat :

L'activité inhibitrice des produits de l'invention est déterminée par la mesure de leur effets sur la transformation par la NO synthase de la [³H]L-arginine en [³H]L-citrulline en accord avec la méthode modifiée de Bredt et Snyder (*Proc. Natl*. *Acad. Sci*. *USA,* (1990) 87: 682-685). Des cervelets de rats Sprague-Dawley (300g - Charles River) sont rapidement prélevés, disséqués à 4 °C et homogénéisés dans un volume de tampon d'extraction (HEPES 50 mM, EDTA 1 mM, pH 7,4, pepstatin A 10 mg/ml, leupeptine 10 mg/ml). Les homogénats sont ensuite centrifugés à 21000 g pendant 15 min à 4 °C. Le dosage se fait dans des tubes à essai en verre dans lesquels sont distribués 100 µl de tampon d'incubation contenant 100 mM d'HEPES (pH 7,4), 2 mM d'EDTA, 2,5 mM de CaCl₂, 2 mM de dithiotréitol, 2 mM de NADPH réduit et 10 µg/ml de calmoduline. On ajoute 25 µl d'une solution contenant 100 nM de [³H]L-arginine (Activité spécifique : 56.4 Ci/mmole, Amersham) et 40 µM de L-arginine non radioactive. La réaction est initiée en ajoutant 50 µl d'homogénat, le volume final étant de 200 µl (les 25 µl manquants sont soit de l'eau, soit le produit testé. Après 15 min, la réaction est stoppée avec 2 ml de tampon d'arrêt (20 mM d'HEPES, pH 5,5, 2 mM d'EDTA). Après passage des échantillons sur une colonne de 1 ml de résine DOWEX, la radioactivité est quantifiée par un spectromètre à scintillation liquide.

Les composés des exemples 1 à 6, 11, 12, 14 et 16 décrits ci-dessus présentent une CI₅₀ inférieure à 4,5 µM.

### Etude des effets sur le stress oxidatif induit par le glutamate sur des cellules en culture (HT-22) :

L'activité inhibitrice des produits de l'invention est déterminée par la mesure de leur capacité à protéger les cellules d'une lignée hippocampale de souris (HT-22) d'un stress oxidatif provoqué par le glutamate. La biosynthèse de glutation, élément essentiel de détoxification cellulaire des radicaux libres, nécessite le transport actif de cystine à l'intérieur de la cellule. Le glutamate en s'opposant à la pénétration de la cystine provoque une réduction du taux de gluthation qui conduit à la mort de la cellule par stress oxidatif (Demerlé-Pallardy, C. et coll., *J*. *Neurochem*. (2000), **74,** 2079-2086 ; Davis, J.B. and Maher, P., *Brain Res*., (1994) **652:** 169-173; Murphy, T.H. et al., *Neuron*, (1989) **2:** 1547-1558). Les cellules sont cultivées à 37 °C dans un milieu DMEM additionné de 10% de sérum de veau foetal. Les essais sont réalisés dans des plaques 96 puits contenant 5000 cellules par puits. Le glutamate (5 mM) est ajouté au milieu contenant ou non les produits à tester. La viabilité cellulaire est testée après 24 h par la méthode du MTT (Hansen, M.B. et al., *J Immunol*.*Methods* (1989) **119:** 203-210). La capacité des composés à protéger les cellules de l'action toxique du glutamate est estimée en CE₅₀, calculée par rapport à la viabilité de cellules non soumises à l'action du glutamate considérée comme viabilité 100%.

Les composés des exemples 1, 2, 4, 5 et 7 à 11 décrits ci-dessus présentent une CE₅₀ inférieure ou égale à 4 µM.

## Revendications

1. Produit de formule générale **(I)**, **caractérisée en ce qu'**elle comprend les produits de sous-formules **(I)a** et **(I)b** 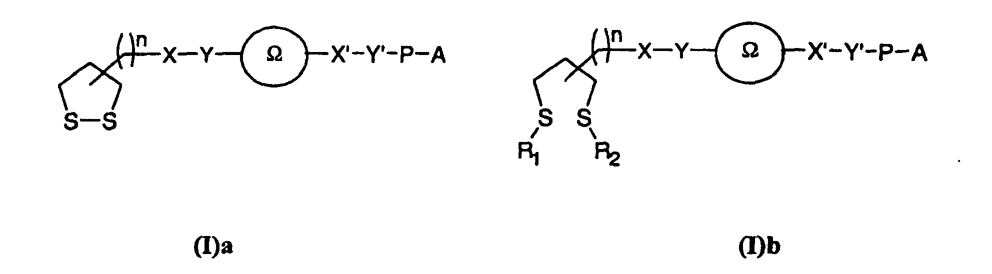 dans lesquelles
n représente un entier de 0 à 6 ;
R₁ et R₂ représentent, indépendamment, un atome d'hydrogène ou un radical alkyle linéaire ou ramifié ayant de 1 à 6 atomes de carbone ;
X-Y représente -O-(CH₂)ᵣ-, -N(R₃)-(CH₂)ᵣ-, -CO(CH₂)ᵣ-, -CO-N(R₃)-(CH₂)ᵣ-, -N(R₄)-CO-(CH₂)ᵣ-, ou -N(R₃)-CO-N(R₄)-(CH₂)ᵣ-,
X'-Y' représente -(CH₂)ᵣ-, -(CH₂)ᵣ-O-(CH₂)ᵣ-, -(CH₂)ᵣ-N(R₃)-(CH₂)ᵣ-, -(CH₂)ᵣ-CO(CH₂)ᵣ-, -(CH₂)ᵣ-CO-N(R₃)-(CH₂)ᵣ-, -(CH₂)ᵣ-N(R₄)-CO-(CH₂)ᵣ- ou -(CH₂)ᵣ-N(R₃)-CO-N(R₄)-(CH₂)ᵣ-,
R₃ et R₄ représentent, indépendamment à chaque fois qu'ils interviennent, un atome d'hydrogène ou un radical alkyle linéaire ou ramifié comptant de 1 à 6 atomes de carbone, un radical alkoxycarbonyle dont le radical alkoxy est linéaire ou ramifié et compte de 1 à 6 atomes de carbone ou un radical aralkoxycarbonyle dont le radical aryle est le radical phényle et le radical alkoxy est linéaire ou ramifié et compte de 1 à 6 atomes de carbone ;
r représentant indépendamment à chaque fois qu'il intervient un entier de 0 à 6 ;
Ω représente un hétérocycle aromatique à 5 ou 6 chaînons, un hétérocycle non aromatique de 4 à 7 chaînons ou un radical phénylène substitué par un radical R₅, R₅ représentant un atome d'hydrogène, un radical alkyle linéaire ou ramifié de 1 à 6 atomes de carbone ou un radical -(CH₂)ₘ-Q dans lequel Q représente un atome halogène ou un radical hydroxy, cyano, amino, alkoxy linéaire ou ramifié et comptant de 1 à 6 atomes de carbone, alkylamino dont le radical alkyle est linéaire ou ramifié et compte de 1 à 6 atomes de carbone ou dialkylamino dont chacun des radicaux alkyle est linéaire ou ramifié et compte de 1 à 6 atomes de carbone, m représentant un entier de 0 à 6 ;
P représente un radical 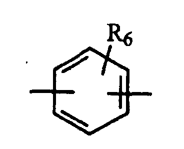 dans lequel R₆ représente un atome d'hydrogène, un radical alkyle linéaire ou ramifié ayant de 1 à 6 atomes de carbone ou un radical -(CH₂)ₙ-Q' dans lequel Q' représente un atome halogène ou un radical trifluorométhyle, hydroxy, amino, cyano, alkoxycarbonylamino dont le radical alkoxy est linéaire ou ramifié et compte de 1 à 6 atomes de carbone, aralkoxycarbonylamino dont le radical aryle est le radical phényle et le radical alkoxy est linéaire ou ramifié et compte de 1 à 6 atomes de carbone, alkoxy linéaire ou ramifié et comptant de 1 à 6 atomes de carbone, alkylthio dont le radical alkyle est linéaire ou ramifié et compte de 1 à 6 atomes de carbone, alkylamino dont le radical alkyle est linéaire ou ramifié et compte de 1 à 6 atomes de carbone ou dialkylamino dont chacun des radicaux alkyle est linéaire ou ramifié et compte de 1 à 6 atomes de carbone et n représente un entier de 0 à 6, ;
et enfin A représente le radical 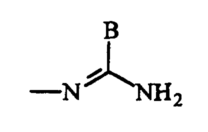 dans lequel :
B représente un radical alkyle linéaire ou ramifié ayant de 1 à 6 atomes de carbone, un radical aryle carbocyclique ou hétérocyclique choisi parmi le groupe constitué par les radicaux phényle, thiophène, furanne, pyrrole et thiazole, ledit radical aryle étant éventuellement substitué par un ou plusieurs groupes choisis parmi les radicaux alkyle, alkényle ou alkoxy linéaires ou ramifiés ayant de 1 à 6 atomes de carbone,
ou B représente NR₈R₉, dans lequel R₈ et R₉ représentent, indépendamment, un atome d'hydrogène ou un radical alkyle linéaire ou ramifié ayant de 1 à 6 atomes de carbone,
ou l'un de R₈ et R₉ représente un radical nitro tandis que l'autre représente un atome d'hydrogène ou un radical alkyle linéaire ou ramifié ayant de 1 à 6 atomes de carbone,
ou encore R₈ et R₉ pris ensemble forment avec l'atome d'azote un hétérocycle non aromatique de cinq à six chaînons, les éléments de la chaîne étant choisis parmi un groupe composé de -CH₂-, -NH-, -O- ou -S-,
ou encore B représente un radical SR₁₀ dans lequel R₁₀ représente un atome d'hydrogène ou un radical alkyle linéaire ou ramifié ayant de 1 à 6 atomes de carbone ;
ou un sel d'un produit de formule générale **(I)**.

2. Produit de formule générale **(I)** telle que définie dans la revendication 1, **caractérisé en ce que** A représente le radical 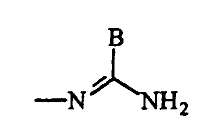 dans lequel B représente un radical thiényle, furannyle ou pyrrolyle ;
ou sel d'un tel produit.

3. Produit de formule générale **(I)** telle que définie dans la revendication 1, **caractérisé en ce que** Ω représente un radical pipérazinyle, pipéridyle ou phényle ;
ou sel d'un tel produit.

4. Produit de formule générale **(I)** telle que définie dans la revendication 1, **caractérisé en ce qu'**il répond à la sous-formule générale **(I)a** de cette même revendication ;
ou sel d'un tel produit.

5. Produit selon la revendication 1, **caractérisé en ce qu'**il s'agit d'un des composés suivants :
- N'-(4-{4-[5-(1,2-dithiolan-3-yl)pentanoyl]-1-pipérazinyl}phényl)-2-thiophènecarboximidamide ;
- N'-(4-{4-[2-(1,2-dithiolan-3-yl)acétyl]-1-pipérazinyl}phényl)-2-thiophènecarboximidamide ;
- N'-[3-({4-[5-(1,2-dithiolan-3-yl)pentanoyl]-1-pipérazinyl}méthyl)phényl]-2-thiophènecarboximidamide ;
- N'-[3-({4-[2-(1,2-dithiolan-3-yl)acétyl]-1-pipérazinyl}méthyl)phényl]-2-thiophènecarboximidamide ;
- 4-(4-{[amino(2-thiényl)méthylidène]amino}phényl)-N-[4-(1,2-dithiolan-3-yl)butyl]-1-pipérazinecarboxamide ;
- 4-(4-{[amino(2-thiényl)méthylidène]amino}-2-méthylphényl)-N-[4-(1,2-dithiolan-3-yl)butyl]-1-pipérazinecarboxamide ;
- 4-(4-{[amino(2-thiényl)méthylidène]amino}-3-méthylphényl)-N-[4-(1,2-dithiolan-3-yl)butyl]-1-pipérazinecarboxamide ;
- 4-(4-{[amino(2-thiényl)méthylidène]amino}-2-méthoxyphényl)-N-[4-(1,2-dithiolan-3-yl)butyl]-1-pipérazinecarboxamide ;
- 4-(4-{[amino(2-thiényl)méthylidène]amino}-3-méthoxyphényl)-N-[4-(1,2-dithiolan-3-yl)butyl]-1-pipérazinecarboxamide ;
- 4-(4-{[amino(2-thiényl)méthylidène]amino}-2-cyanophényl)-N-[4-(1,2-dithiolan-3-yl)butyl]-1-pipérazinecarboxamide ;
- 4-(3-{[amino(2-thiényl)méthylidène]amino}benzyl)-N-[4-(1,2-dithiolan-3-yl)butyl]-1-pipérazinecarboxamide ;
- N'-(3-{4-[5-(1,2-dithiolan-3-yl)pentanoyl]-1-pipérazinyl}phényl)-2-thiophènecarboximidamide ;
- 5-{[amino(2-thiényl)méthylidène]amino}-2-{4-[5-(1,2-dithiolan-3-yl)pentanoyl]-1-pipérazinyl}benzylcarbamate de *tert*-butyle ;
- N'-(3-(aminométhyl)-4-{4-[5-(1,2-dithiolan-3-yl)pentanoyl]-1-pipérazinyl}phényl)-2-thiophènecarboximidamide ;
- 3-{[-amino(2-thiényl)méthylidène]amino}benzyl{1-[5-(1,2-dithiolan-3-yl)pentanoyl]-4-pipéridinyl}carbamate de tert-butyle ;
- N'-{3-[({1-[5-(1,2-dithiolan-3-yl)pentanoyl]-4-pipéridinyl}amino)méthyl]phényl}-2-thiophènecarboximidamide ;
- N-[2-(4-{[amino(2-thiényl)méthylidène]amino}phényl)éthyl]-4-{[5-(1,2-dithiolan-3-yl)pentanoyl]amino}benzamide ;
ou d'un de leurs sels.

6. Procédé de préparation d'un composé de formule générale **(I)** selon la revendication 1, **caractérisé en ce que** l'on fait réagir un composé de formule générale **(II)** 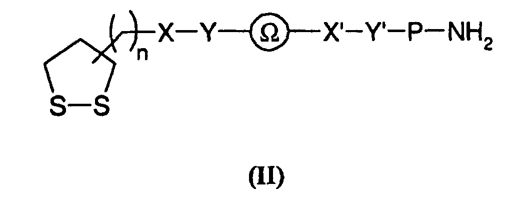 dans laquelle n, X, Y, Ω, X', Y' et P ont la même signification que dans la formule générale **(I)** de la revendication 1,
a) avec le composé de formule générale **(I.i)** 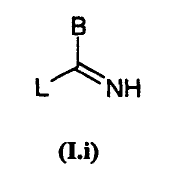 dans laquelle B a la même signification que dans la formule générale **(I)** et L représente un groupe partant, par exemple un radical alkoxy, alkylthio, acide sulfonique, halogénure, alcool arylique ou tosyle,
b) ou avec le composé de formule générale **(I.ii)** 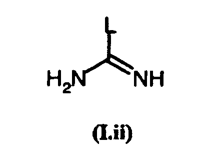 dans laquelle L représente un groupe partant, par exemple un radical alkoxy, alkylthio, acide sulfonique, halogénure, alcool arylique ou tosyle,
c) ou avec le composé de formule générale **(I.iii)** 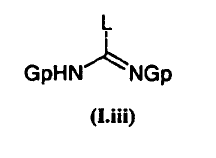 dans laquelle L représente un groupe partant, par exemple un radical alkoxy, alkylthio, acide sulfonique, halogénure, alcool arylique ou tosyle, et Gp un groupe protecteur de type carbamate, par exemple le groupe t-butoxycarbonyle,
cette réaction étant suivie, dans le cas où l'on opte pour la réaction avec le composé de formule générale **(I.iii)**, par une hydrolyse en présence d'un acide fort, par exemple l'acide trifluoroacétique,
d) ou avec le dérivé de formule **(I.iv)** (N-méthyl-N'-nitro-N-nitrosoguanidine) 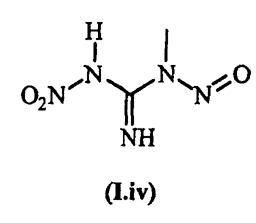
e) ou enfin avec le dérivé de formule **(I.v)** dans laquelle Gp représente un groupe protecteur
S=·=N-Gp. (I.v)

7. Procédé de préparation d'un produit de formule générale **(I)** selon la revendication 1 dans laquelle le groupe X-Y représente -CO-N(R₃)-(CH₂)ᵣ-, **caractérisé en ce que** l'on fait réagir l'acide de formule générale **(I.vi)** 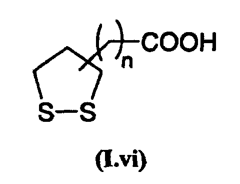 dans laquelle n a la même signification que dans la formule générale **(I)** de la revendication 1, avec une amine de formule générale **(X)** ou **(X*bis*)** 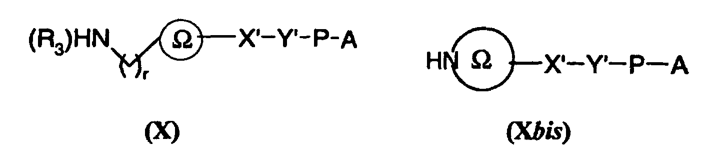 dans lesquelles r, R₃, Ω, X', Y', P et A ont la même signification que dans la formule générale **(I)** de la revendication 1, le composé de formule générale (***Xbis***) étant en outre tel que son hétérocycle Ω comprend un atome d'azote.

8. A titre de produits industriels nouveaux, les intermédiaires de synthèse de formules générales **(II), (III)** et **(IV)** 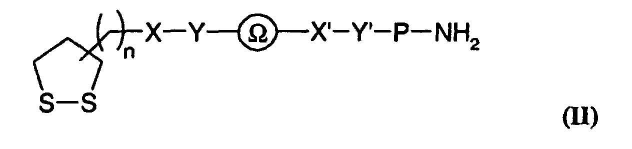 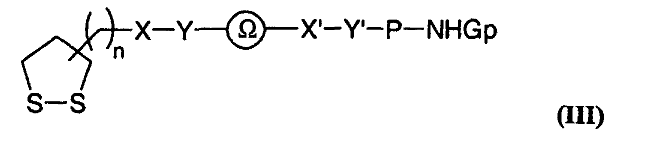 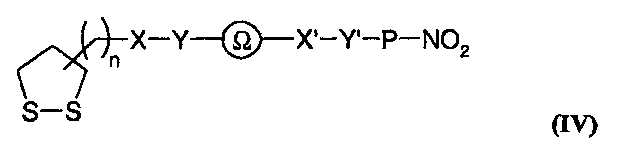 dans lesquelles
n, X', Y', P, A, Ω et X-Y ont la même signification que dans la formule générale **(I)** de la revendication 1 ; et
Gp représente un groupe protecteur de type carbamate, par exemple le groupe t-butoxycarbonyle.

9. A titre de médicament, un produit de formule générale **(I)** selon l'une des revendication 1 à 5, ou un sel pharmaceutiquement acceptable de ce dernier.

10. Composition pharmaceutique contenant à titre de principe actif au moins un produit selon l'une des revendications 1 à 5, ou un sel pharmaceutiquement acceptable dudit produit.

11. Utilisation d'un produit de formule générale **(I)** selon l'une des revendications 1 à 5, ou d'un sel pharmaceutiquement acceptable de ce produit, pour préparer un médicament destiné à inhiber la NO synthase.

12. Utilisation d'un produit de formule générale **(I)** selon l'une des revendications 1 à 5, ou d'un sel pharmaceutiquement acceptable de ce produit, pour préparer un médicament destiné à régénérer des anti-oxydants.

13. Utilisation d'un produit de formule générale **(I)** selon l'un des revendications 1 à 5, ou d'un sel pharmaceutiquement acceptable de ce produit, pour préparer un médicament ayant à la fois une activité d'inhibition de la NO synthase et de régénération d'anti-oxydants.

14. Utilisation d'un produit de formule générale **(I)** selon l'une des revendications 1 à 5, ou d'un sel pharmaceutiquement acceptable de ce produit, pour préparer un médicament destiné à traiter des pathologies dans lesquelles le monoxyde d'azote et / ou le statut rédox des groupements thiols sont impliqués, pathologies telles que les troubles du système nerveux central ou périphérique particulièrement bien représentés par la maladie de Parkinson, les troubles cérébrovasculaires, les maladies prolifératives et inflammatoires, les vomissements, le choc septique, les pathologies résultant des irradiations radioactives, des radiations solaires ou des transplantations d'organes, les maladies autoimmunes et autosomales, les cancer et toutes les pathologies **caractérisées par** une production ou un dysfonctionnement impliquant le monoxyde d'azote et/ou impliquant le statut rédox des groupements thiols.

15. Utilisation d'un produit de formule générale **(I)** selon l'une des revendications 1 à 5, ou d'un sel pharmaceutiquement acceptable de ce produit, pour préparer un médicament destiné à traiter des troubles cérébrovasculaires tels que la migraine, les infarctus cérébraux d'origine ischémique ou hémorragique, les ischémies et les thromboses.

16. Utilisation d'un produit de formule générale **(I)** selon l'une des revendications 1 à 5, ou d'un sel pharmaceutiquement acceptable de ce produit, pour préparer un médicament destiné à traiter les troubles du système nerveux central ou périphériques tels que les maladies neurodégénératives, la douleur et la fibromyalgie, les traumatismes cérébraux ou de la moëlle épinière, le diabète et ses complications incluant les rétinopathies, les néphropathies et les polyneuropathies, l'addiction aux drogues opiacées, à l'alcool et aux substances induisant une accoutumance, les troubles de l'érection et de la reproduction, les désordres cognitifs, les encéphalopathies, la dépression, l'anxiété, la schizophrénie, l'épilepsie, les troubles du sommeil et les troubles alimentaires.

## Claims

1. Product of general formula **(I), characterized in that** it comprises the products of sub-formulae **(I)a** and **(I)b** 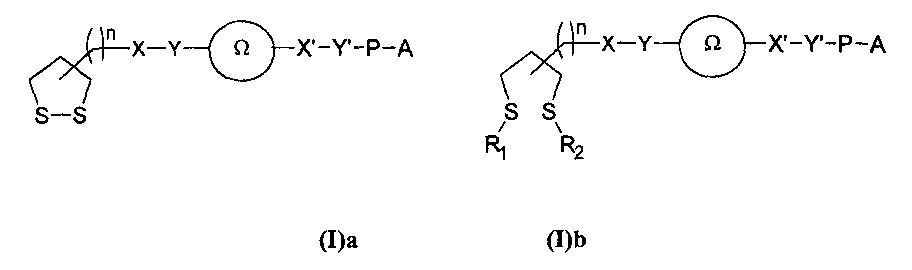 in which
n represents an integer from 0 to 6;
R₁ and R₂ represent, independently, a hydrogen atom or a linear or branched alkyl radical having 1 to 6 carbon atoms;
X-Y represents -O-(CH₂)ᵣ-, -N(R₃)-(CH₂)ᵣ-, -CO(CH₂)ᵣ-, -CO-N(R₃)-(CH₂)ᵣ-, -N(R₄)-CO-(CH₂)ᵣ-, or -N(R₃)-CO-N(R₄)-(CH₂)ᵣ-,
X'-Y' represents -(CH₂)ᵣ-, -(CH₂)ᵣ-O-(CH₂)ᵣ-, -(CH₂)ᵣ-N(R₃)-(CH₂)ᵣ-, -(CH₂)ᵣ-CO(CH₂)ᵣ-, -(CH₂)ᵣ-CO-N(R₃)-(CH₂)ᵣ-, -(CH₂)ᵣ-N(R₄)-CO-(CH₂)ᵣ- or -(CH₂)ᵣ-N(R₃)-CO-N(R₄)-(CH₂)ᵣ-,
R₃ and R₄ represent, independently each time that they occur, a hydrogen atom or a linear or branched alkyl radical containing 1 to 6 carbon atoms, an alkoxycarbonyl radical the alkoxy radical of which is linear or branched and contains 1 to 6 carbon atoms or an aralkoxycarbonyl radical the aryl radical of which is the phenyl radical and the alkoxy radical is linear or branched and contains 1 to 6 carbon atoms;
r representing, independently each time that it occurs, an integer from 0 to 6;
Ω represents an aromatic heterocycle with 5 or 6 members, a non aromatic heterocycle with 4 to 7 members or a phenylene radical substituted by an R₅ radical, R₅ representing a hydrogen atom, a linear or branched alkyl radical with 1 to 6 carbon atoms or a -(CH₂)ₘ-Q radical in which Q represents a halogen atom or a hydroxy, cyano, amino radical, linear or branched alkoxy radical and containing 1 to 6 carbon atoms, alkylamino radial the alkyl radical of which is linear or branched and contains 1 to 6 carbon atoms or dialkylamino radical each of the alkyl radicals of which is linear or branched and contains 1 to 6 carbon atoms, m representing an integer from 0 to 6;
P represents a 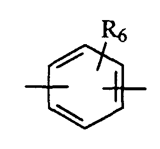 radical in which R₆ represents a hydrogen atom, a linear or branched alkyl radical having 1 to 6 carbon atoms or a -(CH₂)ₙ-Q' radical in which Q' represents a halogen atom or a trifluoromethyl, hydroxy, amino, cyano, alkoxycarbonylamino radical, the alkoxy radical of which is linear or branched and contains 1 to 6 carbon atoms, aralkoxycarbonylamino the aryl radical of which is the phenyl radical and the alkoxy radical is linear or branched and contains 1 to 6 carbon atoms, linear or branched alkoxy and contains 1 to 6 carbon atoms, alkylthio the alkyl radical of which is linear or branched and contains 1 to 6 carbon atoms, alkylamino the alkyl radical of which is linear or branched and contains 1 to 6 carbon atoms or dialkylamino each of the alkyl radicals of which is linear or branched and contains 1 to 6 carbon atoms and n represents an integer from 0 to 6;
and finally A represents the 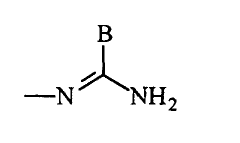 radical in which:
B represents a linear or branched alkyl having 1 to 6 carbon atoms, a carbocyclic or heterocyclic aryl radical chosen from the group constituted by the phenyl, thiophene, furan, pyrrole and thiazole radicals, said aryl radical being optionally substituted by one or more groups chosen from the linear or branched alkyl, alkenyl or alkoxy radicals having 1 to 6 carbon atoms,
or B represents NR₈R₉, in which R₈ and R₉ represent, independently, a hydrogen atom or a linear or branched alkyl radical having 1 to 6 carbon atoms, or one of R₈ and R₉ represents a nitro radical whilst the other represents a hydrogen atom or a linear or branched alkyl radical having 1 to 6 carbon atoms, or also R₈ and R₉ when taken together form, with the nitrogen atom, a non aromatic heterocycle with five to six members, the elements of the chain being chosen from a group comprising -CH₂-, -NH-, -O- or -S-,
or also B represents an SR₁₀ radical in which R₁₀ represents a hydrogen atom or a linear or branched alkyl radical having 1 to 6 carbon atoms;
or a salt of a product of general formula **(I)**.

2. Product of general formula **(I)** as defined in claim 1, **characterized in that** A represents the 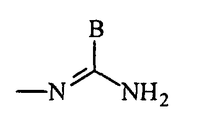 radical, in which B represents a thienyl, furanyl or pyrrolyl radical;
or a salt of such a product.

3. Product of general formula **(I)** as defined in claim 1, **characterized in that** Ω represents a piperazinyl, piperidyl or phenyl radical;
or a salt of such a product.

4. Product of general formula **(I)** as defined in claim 1, **characterized in that** it corresponds to the general sub-formula **(I)a** of this claim;
or a salt of such a product.

5. Product according to claim 1, **characterized in that** it is one of the following compounds:
- N'-(4-{4-[5-(1,2-dithiolan-3-yl)pentanoyl]-1-piperazinyl}phenyl)-2-thiophenecarboximidamide;
- N'-(4- {4-[2-(1,2-dithiolan-3-yl)acetyl]-1-piperazinyl}phenyl)-2-thiophenecarboximidamide;
- N'-[3-({4-[5-(1,2-dithiolan-3-yl)pentanoyl]-1-piperazinyl}methyl)phenyl]-2-thiophenecarboximidamide;
- N'-[3-({4-[2-(1,2-dithiolan-3-yl)acetyl]-1-piperazinyl}methyl)phenyl]-2-thiophenecarboximidamide;
- 4-(4-{[amino(2-thienyl)methylidene]amino}phenyl)-N-[4-(1,2-dithiolan-3-yl)butyl]-1-piperazinecarboxamide;
- 4-(4-{[amino(2-thienyl)methylidene]amino}-2-methylphenyl)-N-[4-(1,2-dithiolan-3-yl)butyl]-1-piperazinecarboxamide;
- 4-(4-{[amino(2-thienyl)methylidene]amino}-3-methylphenyl)-N-[4-(1,2-dithiolan-3-yl)butyl]-1-piperazinecarboxamide;
- 4-(4-{[amino(2-thienyl)methylidene]amino}-2-methoxyphenyl)-N-[4-(1,2-dithiolan-3-yl)butyl]-1-piperazinecarboxamide;
- 4-(4-{[amino(2-thienyl)methylidene]amino}-3-methoxyphenyl)-N-[4-(1,2-dithiolan-3-yl)butyl]-1-piperazinecarboxamide;
- 4-(4-{[amino(2-thienyl)methylidene]amino}-2-cyanophenyl)-N-[4-(1,2-dithiolan-3-yl)butyl]-1-piperazinecarboxamide;
- 4-(3-{[amino(2-thienyl)methylidene]amino}benzyl)-N-[4-(1,2-dithiolan-3-yl)butyl]-1-piperazinecarboxamide;
- N'-(3-{4-[5-(1,2-dithiolan-3-yl)pentanoyl]-1-piperazinyl}phenyl)-2-thiophenecarboximidamide;
- *tert*-butyl 5-{[amino(2-thienyl)methylidene]amino}-2-{4-[5-(1,2-dithiolan-3-yl) pentanoyl]-1-piperazinyl}benzylcarbamate;
- N'-(3-(aminomethyl)-4-{4-[5-(1,2-dithiolan-3-yl)pentanoyl]-1-piperazinyl}phenyl)-2-thiophenecarboximidamide;
- tert-butyl 3-{[-amino(2-thienyl)methylidene]amino}benzyl{1-[5-(1,2-dithiolan-3-yl)pentanoyl]- 4-piperidinyl}carbamate;
- N'-{3-[({1-[5-(1,2-dithiolan-3-yl)pentanoyl]-4-piperidinyl}amino)methyl]phenyl}-2-thiophenecarboximidamide;
- N-[2-(4-{[amino(2-thienyl)methylidene]amino}phenyl)ethyl]-4-{[5-(1,2-dithiolan-3-yl)pentanoyl]amino}benzamide;
or one of their salts.

6. Process for the preparation of a compound of general formula **(I)** according to claim 1, **characterized in that** a compound of general formula **(II)** 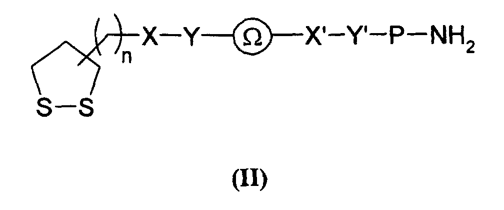 in which n, X, Y, Ω, X', Y' and P have the same meaning as in general formula **(I)** of claim 1 is reacted,
a) with the compound of general formula **(I.i)** 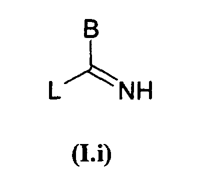 in which B has the same meaning as in general formula **(I)** and L represents a leaving group, for example an alkoxy, alkylthio, sulphonic acid, halide, aryl alcohol or tosyl radical,
b) or with the compound of general formula **(I.ii)** 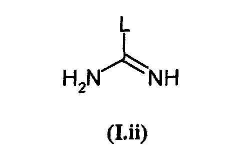 in which L represents a leaving group, for example an alkoxy, alkylthio, sulphonic acid, halide, aryl alcohol or tosyl radical,
c) or with the compound of general formula **(I.iii)** 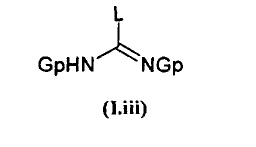 in which L represents a leaving group, for example an alkoxy, alkylthio, sulphonic acid, halide, aryl alcohol or tosyl radical, and Gp a protective group of carbamate type, for example the t-butoxycarbonyl group,
this reaction being followed, in the case where the reaction with the compound of general formula **(I.iii)** is chosen, by hydrolysis in the presence of a strong acid, for example trifluoroacetic acid,
d) or with the derivative of formula **(I.iv)** (N-methyl-N'-nitro-N-nitrosoguanidine) 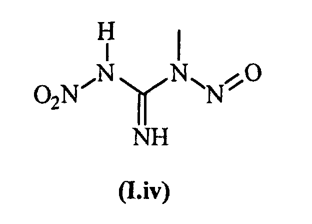
e) or finally with the derivative of formula **(I.v)** in which Gp represents a protective group
S=·=N-Gp. (I.v)

7. Process for the preparation of a product of general formula **(I)** according to claim 1 in which the X-Y group represents -CO-N(R₃)-(CH₂)ᵣ-, **characterized in that** the acid of general formula **(I.vi)** 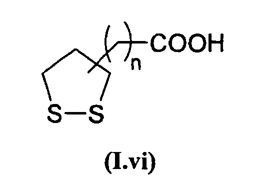 in which n has the same meaning as in the general formula **(I)** of claim 1, is reacted with an amine of general formula **(X)** or **(X*a)*** 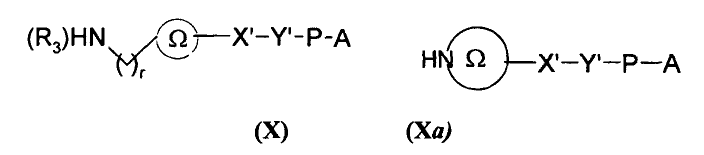 in which r, R₃, Ω, X', Y', P and A have the same meaning as in the general formula **(I)** of claim 1, the compound of general formula **(X*a*)** being moreover such that its Ω heterocycle comprises a nitrogen atom.

8. As new industrial products, the synthesis intermediates of general formulae **(II)**, **(III)** and **(IV)** 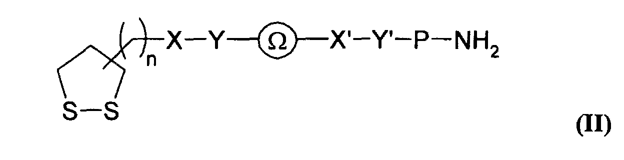 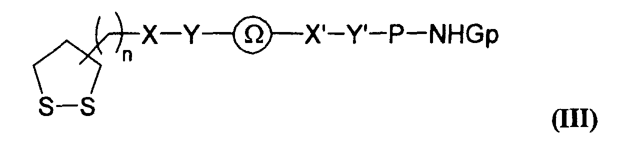 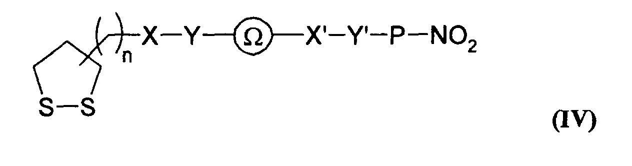 in which
n, X', Y', P, A, Ω and X-Y have the same meaning as in the general formula **(I)** of claim 1; and
Gp represents a protective group of carbamate type, for example the t-butoxycarbonyl group;

9. As a medicament, a product of general formula **(I)** according to one of claims 1 to 5, or a pharmaceutically acceptable salt of the latter.

10. Pharmaceutical composition containing as active ingredient at least one product according to one of claims 1 to 5, or a pharmaceutically acceptable salt of said product.

11. Use of a product of general formula **(I)** according to one of claims 1 to 5, or of a pharmaceutically acceptable salt of this product, for preparing a medicament intended to inhibit NO synthase.

12. Use of a product of general formula **(I)** according to one of claims 1 to 5, or of a pharmaceutically acceptable salt of this product, for preparing a medicament intended to regenerate anti-oxidants.

13. Use of a product of general formula **(I)** according to one of claims 1 to 5, or of a pharmaceutically acceptable salt of this product, for preparing a medicament having both an activity of inhibiting NO synthase and regeneration of anti-oxidants.

14. Use of a product of general formula **(I)** according to one of claims 1 to 5, or a pharmaceutically acceptable salt of this product, for preparing a medicament intended to treat pathologies in which nitrogen monoxide and / or the redox status of the thiol groups are involved, pathologies such as disorders of the central or peripheral nervous system particularly well represented by Parkinson's disease, cerebro-vascular disorders, proliferative and inflammatory diseases, vomiting, septic shock, pathologies resulting from radioactive irradiation, solar radiation or organ transplantations, auto-immune and autosomal diseases, cancer and all the pathologies **characterised by** a production or a dysfunction involving nitrogen monoxide and/or involving the redox status of the thiol groups.

15. Use of a product of general formula **(I)** according to one of claims 1 to 5, or of a pharmaceutically acceptable salt of this product, for preparing a medicament intended to treat cerebro-vascular disorders such as migraine, cerebral infarction of ischemic or hemorragic origin, ischemias and thromboses.

16. Use of a product of general formula **(I)** according to one of claims 1 to 5, or of a pharmaceutically acceptable salt of this product, for preparing a medicament intended to treat disorders of the central or peripheral nervous system such as neurodegenerative diseases, pain and fibromyalgia, cerebral and spinal cord trauma, diabetes and its complications including retinopathies, nephropathies and polyneuropathies, addiction to opiates, alcohol and addictive substances, erective and reproductive disorders, cognitive disorders, encephalopathies, depression, anxiety, schizophrenia, epilepsy, sleeping disorders and eating disorders.

## Patentansprüche

1. Produkt der allgemeinen Formel (I), **dadurch gekennzeichnet, daß** es die Produkte der Unterformeln (I)a und (I)b 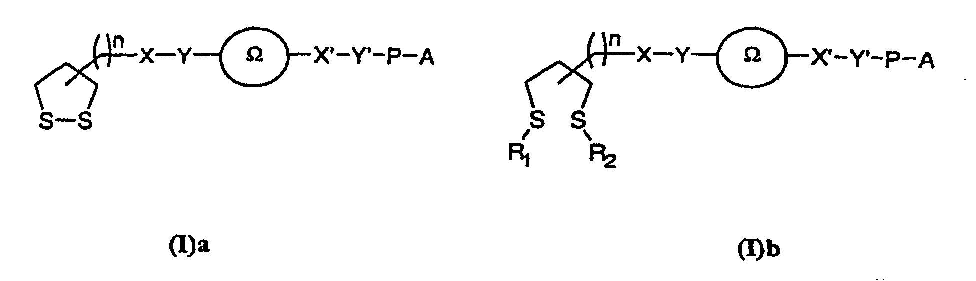 umfaßt, in denen
n eine ganze Zahl von 0 bis 6 darstellt;
R₁ und R₂ unabhängig voneinander ein Wasserstoffatom oder einen linearen oder verzweigten Alkylrest mit 1 bis 6 Kohlenstoffatomen darstellen;
X-Y darstellt: -O-(CH₂)ᵣ-, -N(R₃)-(CH₂)ᵣ-, -CO(CH₂)ᵣ-, -CO-N(R₃)-(CH₂)ᵣ-, -N(R₄)-CO-(CH₂)ᵣ- oder -N(R₃)-CO-N(R₄)-(CH₂)ᵣ-,
X'-Y' darstellt: -(CH₂)ᵣ-, -(CH₂)ᵣ-O-(CH₂)ᵣ-, -(CH₂)ᵣ-N(R₃)-(CH₂)ᵣ-, -(CH₂)ᵣ-CO(CH₂)ᵣ-, -(CH₂)ᵣ-CO-N(R₃)-(CH₂)ᵣ-, -(CH₂)ᵣ-N(R₄)-CO-(CH₂)ᵣ- oder -(CH₂)ᵣ-N(R₃)-CO-N(R₄)-(CH₂)ᵣ-,
R₃ und R₄ jedesmal, wenn sie auftreten, unabhängig voneinander ein Wasserstoffatom oder einen linearen oder verzweigten Alkylrest mit 1 bis 6 Kohlenstoffatomen, einen Alkoxycarbonylrest, dessen Alkoxyrest linear oder verzweigt ist und 1 bis 6 Kohlenstoffatome aufweist, oder einen Aralkoxycarbonylrest darstellen, dessen Arylrest ein Phenylrest ist und dessen Alkoxyrest linear oder verzweigt ist und 1 bis 6 Kohlenstoffatome aufweist;
r jedesmal, wenn es auftritt, unabhängig voneinander eine ganze Zahl von 0 bis 6 darstellt;
Ω einen aromatischen Heterocyclus mit 5 oder 6 Gliedern, einen nicht aromatischen Heterocyclus mit 4 bis 7 Gliedern oder einen durch einen Rest R₅ substituierten Phenylenrest darstellt, wobei R₅ ein Wasserstoffatom, einen linearen oder verzweigten Alkylrest mit 1 bis 6 Kohlenstoffatomen oder einen Rest -(CH₂)ₘ-Q darstellt, in dem Q ein Halogenatom oder einen der Reste Hydroxy, Cyan, Amino, lineares oder verzweigtes Alkoxy mit 1 bis 6 Kohlenstoffatomen, Alkylamino, dessen Alkylrest linear oder verzweigt ist und 1 bis 6 Kohlenstoffatome aufweist, oder Dialkylamino, bei dem jeder der Alkylreste linear oder verzweigt ist und 1 bis 6 Kohlenstoffatome aufweist, darstellt, wobei m eine ganze Zahl von 0 bis 6 darstellt;
P einen Rest 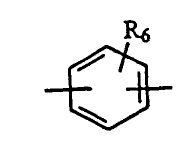 darstellt, in dem R₆ ein Wasserstoffatom, einen linearen oder verzweigten Alkylrest mit 1 bis 6 Kohlenstoffatomen oder einen Rest -(CH₂)ₙ-Q' darstellt, worin Q' ein Halogenatom oder einen der Reste Trifluormethyl, Hydroxy, Amino, Cyan, Alkoxycarbonylamino, dessen Alkoxyrest linear oder verzweigt ist und 1 bis 6 Kohlenstoffatome aufweist, Aralkoxycarbonylamino, dessen Arylrest ein Phenylrest ist und dessen Alkoxyrest linear oder verzweigt ist und 1 bis 6 Kohlenstoffatome aufweist, lineares oder verzweigtes Alkoxy mit 1 bis 6 Kohlenstoffatomen, Alkylthio, dessen Alkylrest linear oder verzweigt ist und 1 bis 6 Kohlenstoffatome aufweist, Alkylamino, dessen Alkylrest linear oder verzweigt ist und 1 bis 6 Kohlenstoffatome aufweist, oder Dialkylamino, bei dem jeder der Alkylreste linear oder verzweigt ist und 1 bis 6 Kohlenstoffatome aufweist, darstellt und n eine ganze Zahl von 0 bis 6 darstellt;
und A den Rest 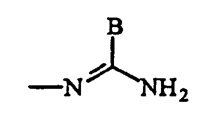 darstellt, in dem:
B einen linearen oder verzweigten Alkylrest mit 1 bis 6 Kohlenstoffatomen, einen carbocyclischen oder heterocyclischen Arylrest darstellt, der aus der Gruppe ausgewählt ist, die aus den Resten Phenyl, Thiophen, Furan, Pyrrol und Thiazol besteht, wobei dieser Arylrest ggf. durch eine oder mehrere Gruppen substituiert ist, die aus den linearen oder verzweigten Resten Alkyl, Alkenyl oder Alkoxy mit 1 bis 6 Kohlenstoffatomen ausgewählt sind,
oder B NR₈R₉ darstellt, worin R₈ und R₉ unabhängig voneinander ein Wasserstoffatom oder einen linearen oder verzweigten Alkylrest mit 1 bis 6 Kohlenstoffatomen darstellen oder einer der Reste R₈ und R₉ einen Nitrorest darstellt, während der andere ein Wasserstoffatom oder einen linearen oder verzweigten Alkylrest mit 1 bis 6 Kohlenstoffatomen darstellt, oder R₈ und R₉ zusammen mit dem Stickstoffatom einen nicht aromatischen Heterocyclus von fünf bis sechs Kettengliedern bilden, wobei die Elemente der Kette aus einer Gruppe ausgewählt sind, die aus -CH₂-, NH-, -O- oder -S- besteht,
oder B einen Rest SR₁₀ darstellt, worin R₁₀ ein Wasserstoffatom oder einen linearen oder verzweigten Alkylrest mit 1 bis 6 Kohlenstoffatomen darstellt;
oder ein Salz eines Produkts der allgemeinen Formel (I).

2. Produkt der allgemeinen Formel (I), wie sie in Anspruch 1 definiert ist, **dadurch gekennzeichnet, daß** A den Rest 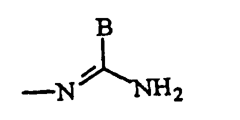 darstellt, in dem B einen Thienyl-, Furanyl oder Pyrrolylrest darstellt;
oer ein Salz eines solchen Produkts.

3. Produkt der allgemeinen Formel (I), wie sie in Anspruch 1 definiert ist, **dadurch gekennzeichnet, daß** Ω einen Piperazinyl-, Piperidyl- oder Phenylrest darstellt;
oder ein Salz eines solchen Produkts.

4. Produkt der allgemeinen Formel (I), wie sie in Anspruch 1 definiert ist, **dadurch gekennzeichnet, daß** es der allgemeinen Unterformel (I)a desselben Anspruchs entspricht;
oder ein Salz eine solchen Produkts.

5. Produkt nach Anspruch 1, **dadurch gekennzeichnet, daß** es sich um eine der folgenden Verbindungen handelt:
- N'-(4-{4-[5-(1,2-Dithiolan-3-yl)pentanoyl]-1-piperazinyl}phenyl)-2-thiophencarboximidamid;
- N'-(4-{4-[2-(1,2-Dithiolan-3-yl)acetyl]-1-piperazinyl}phenyl)-2-thiophencarboximidamid;
- N'-[3-({4-[5-(1,2-Dithiolan-3-yl)pentanoyl]-1-piperazinyl}methyl)phenyl]-2-thiophencarboximidamid;
- N'-[3-({4-[2-(1,2-Dithiolan-3-yl)acetyl]-1-piperazinyl}methyl)phenyl]-2-thiophencarboximidamid;
- 4-(4-{[Amino(2-thienyl)methyliden]amino}phenyl)-N-[4-(1,2-dithiolan-3-yl)butyl]-1-piperazincarboxamid;
- 4-(4-{[Amino(2-thienyl)methyliden]amino}-2-methylphenyl)-N-[4-(1,2-dithiolan-3-yl)butyl]-1-piperazincarboxamid;
- 4-(4-{[Amino(2-thienyl)methyliden]amino}-3-methylphenyl)-N-[4-(1,2-dithiolan-3-yl)butyl]-1-piperazincarboxamid;
- 4-(4-{[Amino(2-thienyl)methyliden]amino}-2-methoxyphenyl)-N-[4-(1,2-dithiolan-3-yl)butyl]-1-piperazincarboxamid;
- 4-(4-{[Amino(2-thienyl)methyliden]amino}-3-methoxyphenyl)-N-[4-(1,2-dithiolan-3-yl)butyl]-1-piperazincarboxamid;
- 4-(4-{[Amino(2-thienyl)methyliden]amino}-2-cyanophenyl)-N-[4-(1,2-dithiolan-3-yl)butyl]-1-piperazincarboxamid;
- 4-(3-{[Amino(2-thienyl)methyliden]amino}benzyl)-N-[4-(1,2-dithiolan-3-yl)butyl]-1-piperazincarboxamid;
- N'-(3-{4-[5-(1,2-Dithiolan-3-yl)pentanoyl]-1-piperazinyl}phenyl)-2-thiophencarboximidamid;
- 5-{[Amino(2-thienyl)methyliden]amino}-2-{4-[5-(1,2-dithiolan-3-yl)pentanoyl]-1-piperazinyl}*tert*-butylbenzylcarbamat;
- N'-(3-(Aminomethyl)-4-{4-[5-(1,2-dithiolan-3-yl)pentanoyl]-1-piperazinyl}phenyl)-2-thiophencarboximidamid;
- 3-{[-Amino(2-thienyl)methyliden]amino}benzyl{1-[5-(1,2-dithiolan-3-yl)pentanoyl]-4-piperidinyl}*tert*-butylcarbamat;
- N'-{3-[({1-[5-(1,2-Dithiolan-3-yl)pentanoyl]-4-piperidinyl}amino)methyl]phenyl}-2-thiophencarboximidamid;
- N-[2-(4-{[Amino(2-thienyl)methyliden]amino}phenyl) ethyl]-4-{[5-(1,2-dithiolan-3-yl)pentanoyl]amino}benzamid;
oder um eines ihrer Salze.

6. Verfahren zur Herstellung einer Verbindung der allgemeinen Formel (I) nach Anspruch 1, **dadurch gekennzeichnet, daß** man eine Verbindung der allgemeinen Formel (II) 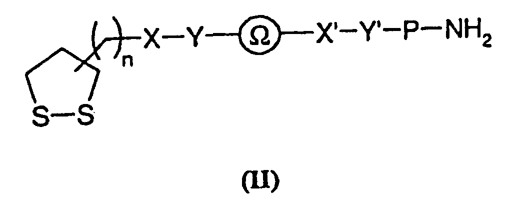 in der n, X, Y, Ω, X', Y' und P dieselbe Bedeutung wie in der allgemeinen Formel (I) des Anspruchs 1 haben,
reagieren läßt:
a) mit der Verbindung der allgemeinen Formel (I.i) 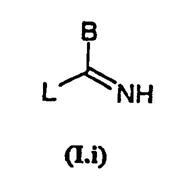 in der B dieselbe Bedeutung wie in der allgemeinen Formel (I) hat und L eine Abgangsgruppe, beispielsweise einen der Reste Alkoxy, Alkylthio, Sulfonsäure, Halogenid, Arylalkohol oder Tosyl, darstellt,
b) oder mit der Verbindung der allgemeinen Formel (I.ii) 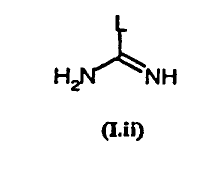 in der L eine Abgangsgruppe, beispielsweise einen der Reste Alkoxy, Alkylthio, Sulfonsäure, Halogenid, Arylalkohol oder Tosyl, darstellt,
c) oder mit der Verbindung der allgemeinen Formel (I.iii) 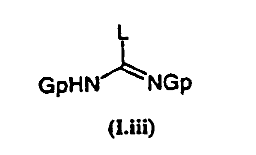 in der L eine Abgangsgruppe, beispielsweise einen der Reste Alkoxy, Alkylthio, Sulfonsäure, Halogenid, Arylalkohol oder Tosyl, und Gp eine Schutzgruppe vom Typ Carbamat, beispielsweise die t-Butoxycarbonyl-Gruppe, darstellen,
wobei auf diese Reaktion, wenn es sich dabei um die Reaktion mit der Verbindung der allgemeinen Formel (I.iii) handelt, eine Hydrolyse in Gegenwart einer starken Säure, beispielsweise Trifluoressigsäure, folgt,
d) oder mit dem Derivat der Formel (I.iv) (N-Methyl-N'nitro-N-nitrosoguanidin) 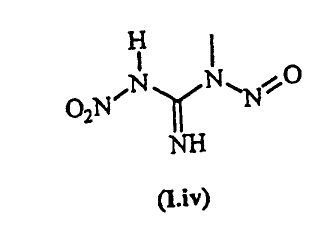
e) oder mit dem Derivat der Formel (I.v)
S=·=N-Gp. (I.v)
worin Gp eine Schutzgruppe darstellt.

7. Verfahren zur Herstellung eines Produkts der allgemeinen Formel (I) nach Anspruch 1, in der die Gruppe X-Y -CO-N(R₃)-(CH₂)ᵣ- darstellt, **dadurch gekennzeichnet, daß** man die Säure der allgemeinen Formel (I.vi) 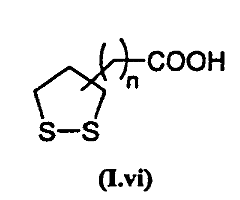 in der n dieselbe Bedeutung wie in der allgemeinen Formel (I) des Anspruchs 1 hat, mit einem Amin der allgemeinen Formel (X) oder (Xbis) reagieren läßt, 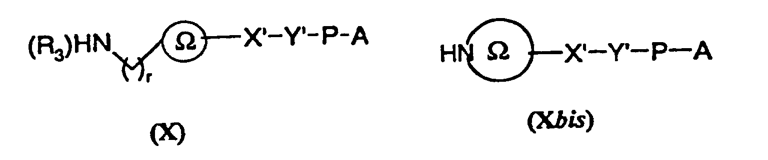 in denen r, R₃, Ω, X', Y', P und A dieselbe Bedeutung wie in der allgemeinen Formel (I) des Anspruchs 1 haben, wobei die Verbindung der allgemeinen Formel (Xbis) außerdem so beschaffen ist, daß ihr Heterocyclus Ω ein Stickstoffatom aufweist.

8. Synthesezwischenprodukte der allgemeinen Formeln (II), (III) und (IV) 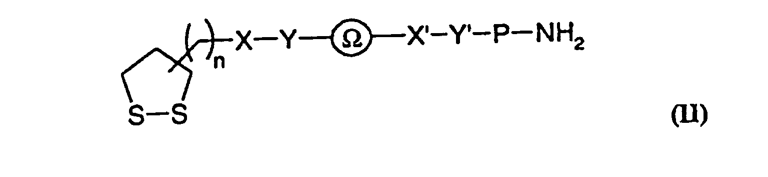 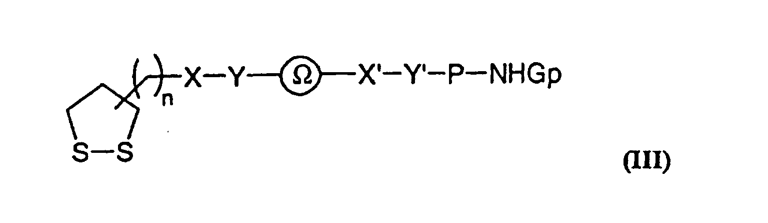 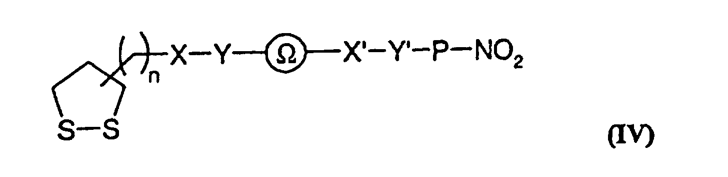 in denen
n, X', Y', P, A, Ω und X-Y dieselbe Bedeutung wie in der allgemeinen Formel (I) des Anspruchs 1 haben; und
Gp eine Schutzgruppe vom Typ Carbamat, beispielsweise die t-Butoxycarbonyl-Gruppe, darstellt,
in Form von neuen Industrieprodukten.

9. Ein Produkt der allgemeinen Formel (I) nach einem der Ansprüche 1 bis 5 oder ein pharmazeutisch verträgliches Salz dieses Produkts in Form eines Medikaments.

10. Pharmazeutische Zusammensetzung, die als Wirkstoff mindestens ein Produkt nach einem der Ansprüche 1 bis 5 oder ein pharmazeutisch verträgliches Salz dieses Produkts enthält.

11. Verwendung eines Produkts der allgemeinen Formel (I) nach einem der Ansprüche 1 bis 5 oder eines pharmazeutisch verträglichen Salzes dieses Produkts zur Herstellung eines zur Hemmung der NO-Synthase bestimmten Medikaments.

12. Verwendung eines Produkts der allgemeinen Formel (I) nach einem der Ansprüche 1 bis 5 oder eines pharmazeutisch verträglichen Salzes dieses Produkts zur Herstellung eines zur Regenerierung von Antioxidantien bestimmten Medikaments.

13. Verwendung eines Produkts der allgemeinen Formel (I) nach einem der Ansprüche 1 bis 5 oder eines pharmazeutisch verträglichen Salzes dieses Produkts zur Herstellung eines Medikaments, das gleichzeitig eine die NO-Synthase hemmende und Antioxidantien regenerierende Wirkung hat.

14. Verwendung eines Produkts der allgemeinen Formel (I) nach einem der Ansprüche 1 bis 5 oder eines pharmazeutisch verträglichen Salzes dieses Produkts zur Herstellung eines Medikaments, das zur Behandlung von Krankheiten bestimmt ist, an denen Stickstoffmonoxid und/oder der Redoxstatus der Thiolgruppen beteiligt sind, Krankheiten, wie z.B. Störungen des zentralen oder peripheren Nervensystems, die insbesondere durch die Parkinson'sche Krankheit vertreten sind, zerebrovaskuläre Störungen, proliferative und entzündliche Krankheiten, Erbrechen, septischer Schock, Krankheiten infolge von radioaktiven Bestrahlungen, Sonnenbestrahlungen oder Organtransplantationen, Autoimmunerkrankungen und autosomale Krankheiten, Krebs und alle Krankheiten, die durch eine Produktion oder eine Dysfunktion gekennzeichnet sind, an der Stickstoffmonoxid und/oder der Redoxstatus der Thiolgruppen beteiligt sind.

15. Verwendung eines Produkts der allgemeinen Formel (I) nach einem der Ansprüche 1 bis 5 oder eines pharmazeutisch verträglichen Salzes dieses Produkts zur Herstellung eines Medikaments, das für die Behandlung von zerebrovaskulären Störungen bestimmt ist, wie Migräne, Zerebralinfarkte ischämischen oder hämorrhagischen Ursprungs, Ischämien und Thrombosen.

16. Verwendung eines Produkts der allgemeinen Formel (I) nach einem der Ansprüche 1 bis 5 oder eines pharmazeutisch verträglichen Salzes dieses Produkts zur Herstellung eines Medikaments, das für die Behandlung von Störungen des zentralen oder peripheren Nervensystems bestimmt ist, wie z.B. neurodegenerative Erkrankungen, Schmerz und Fibromyalgie, Traumatismen des Hirns oder des Rückenmarks, Diabetes und ihre Komplikationen einschließlich Retinopathien, Nephropathien und Polyneuropathien, Abhängigkeit von opiumhaltigen Drogen, Alkohol und eine Gewöhnung erzeugenden Substanzen, Erektions- und Fortpflanzungsstörungen, kognitive Störungen, Enzephalopathien, Depression, Angstzustände, Schizophrenie, Epilepsie, Schlafstörungen und Ernährungsstörungen.
